# EUROPEAN PATENT SPECIFICATION

(11) **EP 4 185 716 B1**
(45) Date of publication and mention of the grant of the patent: **19.02.2025**
(21) Application number: 21755837.8
(22) Date of filing: 22.07.2021
(51) Int. Cl.: C12Q 1/6886

(54) **METHOD FOR IN VITRO DIAGNOSIS OF HEAD AND NECK CANCER AND RELATED KIT**
VERFAHREN ZUR IN-VITRO-DIAGNOSE VON KOPF-HALS-KREBS UND ZUGEHÖRIGES KIT
PROCÉDÉ DE DIAGNOSTIC IN VITRO DE CANCER DE LA TÊTE ET DU COU ET KIT ASSOCIÉ

(30) Priority: 23.07.2020 IT 202000017896
(43) Date of publication of application: 31.05.2023
(73) Proprietor: Eurofins Genoma Group Srl, 00138 Roma (RM) (IT); Istituti Fisioterapici Ospitalieri, 00144 Roma (IT)
(72) Inventor: GANCI, Federica, 00144 Roma (RM) (IT); SPINELLA, Francesca, 00138 Roma (RM) (IT); FIORENTINO, Francesco, 00138 Roma (RM) (IT); BLANDINO, Giovanni, 00144 Roma (RM) (IT)
(74) Representative: Gitto, Serena
(86) International application number: PCT/IT2021/050222
(87) International publication number: WO 2022/018777

(56) References cited:
- WO-A1-2017/077499
- VAN GINKEL JOOST H. ET AL: "Targeted sequencing reveals TP53 as a potential diagnostic biomarker in the post-treatment surveillance of head and neck cancer", ONCOTARGET, vol. 7, no. 38, 11 August 2016 (2016-08-11), pages 61575 - 61586, XP055788451, DOI: 10.18632/oncotarget.11196
- FALLAI ET AL: "Loco-Regionally Advanced Oropharynx Carcinoma Treated With Radiotherapy or Radio-Chemotherapy: Prognostic Value of P53", INTERNATIONAL JOURNAL OF RADIATION: ONCOLOGY BIOLOGY PHYSICS, PERGAMON PRESS, USA, vol. 69, no. 3, 26 October 2007 (2007-10-26), pages S448, XP022316194, ISSN: 0360-3016, DOI: 10.1016/J.IJROBP.2007.07.1618
- RAUDENSKA MARTINA ET AL: "Prognostic significance of the tumour-adjacent tissue in head and neck cancers", TUMOR BIOLOGY, KARGER, BASEL, CH, vol. 36, no. 12, 14 July 2015 (2015-07-14), pages 9929 - 9939, XP036217969, ISSN: 1010-4283, [retrieved on 20150714], DOI: 10.1007/S13277-015-3755-X
- YANG KE ET AL: "Identification of SERPINE1, PLAU and ACTA1 as biomarkers of head and neck squamous cell carcinoma based on integrated bioinformatics analysis", INTERNATIONAL JOURNAL OF CLINICAL ONCOLOGY, CHURCHILL LIVINGSTONE JAPAN, TOKYO, GB, vol. 24, no. 9, 1 April 2019 (2019-04-01), pages 1030 - 1041, XP036856044, ISSN: 1341-9625, [retrieved on 20190401], DOI: 10.1007/S10147-019-01435-9
- CABANILLAS RUBÉN ET AL: "Novel germline CDKN2A mutation associated with head and neck squamous cell carcinomas and melanomas", HEAD AND NECK., vol. 35, no. 3, 15 November 2011 (2011-11-15), US, pages E80 - E84, XP055788471, ISSN: 1043-3074, DOI: 10.1002/hed.21911

## Description

The present invention concerns a method for *in vitro* diagnosis of head and neck cancer and related kit. In particular, the present invention concerns a method of identification of head and neck cancer in tissue or liquid samples in order to provide a diagnosis of primitive cancer, of cancer recurrences and of residual cancer cells in the resection margins of a surgical removal of the head and neck cancer.

It is known that squamous-cell cancer of the head and neck (HNSCC) is the sixth most common malignancy, with 650000 new cases and 350000 HNSCC-related deaths reported annually worldwide (1). The most common anatomic site of HNSCC was the oral cavity (-70%), followed by the larynx and pharynx. HNSCC is typically characterized by loco-regional diffusion and low propensity to develop distant metastasis. Due to the lack of symptoms in the early stage of the disease, about two thirds of patients are diagnosed in advanced stage with lymph node metastases. These advanced tumors are characterized by high recurrence rates that occur in -50-60% of cases and it is the main cause of death in patients affected by HNSCC (1). Of note, one key feature of HNSCC is the insurgence of recurrences after seemingly complete surgical resection, probably due to the existence of preneoplastic processes at multiple sites in the mucosa ("field cancerization" hypothesis) or tumor cells histologically not-detected (Minimal Residual Disease, MRD)(1).

Despite advances in the knowledge on head and neck carcinogenesis and innovations in surgery, chemotherapy and radiation therapy, the survival rate for many types of HNSCC (about five-year survival rate) is still low and have not improved significantly over the past 40 years. This can be attributed to the complex anatomy and histology of the head and neck region, but also to the lack of cost-effective diagnostic systems for early detection of head and neck cancer insurgence as well as efficient prognostic patient monitoring tools.

Indeed, the TNM staging system (TNM Classification of Malignant Tumors), used to classify HNSCC patients, still remains the main system to classify HNSCC patients. However, the TNM staging system does not adequately address the molecular heterogeneity of HNSCC tumors for which patients with the same clinic-pathologic stage do not have the same disease progression, response to therapy, rate of disease recurrence and survival.

Patient management procedures based on tests including imaging (MRI and PET) and tissue examination are expensive and often inconclusive. In particular, disease progression after curative treatment is monitored by clinical evaluation combined with imaging, but their specificity is low due to the post treatment effects; tissue changes due to the radiotherapy could be misinterpreted as evidence of persistent or recurrent tumour (2). Consequently, many recurrences are not identified until an advanced stage has been reached (typically between 9 and 12 months after treatment stop) with a further increasing of patients' mortality and thereby costing the health system more. In particular, for the inoperable or severe metastasis or recurrences diagnosed at late stage, the response rates is only 10-35% and the median survival is 6-12 months.

Current diagnostic methods for HNSCC make challenging the detection of early disease, assessment of response to treatment, and differentiation between the adverse effects of treatment versus persistent or recurrent disease. These issues collectively compromise clinical decision-making and impair patient management.

In addition, it has to be noted that about 10-30% of surgically treated HNSCC patients develop local recurrences despite the resection margins appear histologically tumor-free. This indicates the need to obtain a more detailed molecular characterization to provide a more rational therapeutic approach, potentially relevant to diagnosis and prognosis of HNSCC cancer. Mutation in TP53 tumour suppressor gene is the most frequently detectable genetic alteration (about 70-80%) reported in HNSCC. It is known that the presence of TP53-mutated DNA in the surgical margins was related to the presence of tumor-related precursor lesions in 20% of the cases (3). Additionally it was demonstrated that the probability of developing locoregional recurrence was significantly higher for the group with TP53 mutation-positive margins when compared with the group with clear margins (3).

In the light of the above, there is an urgent and unmet need to develop and to introduce in the clinical practice innovative and predictive diagnostic and prognostic tools for HNSCC. In particular, it is also apparent the need to improve detection of tumor or precancerous cells in histologically negative margins.

Methods used to perform mutational analysis in biological samples from tissue and liquid biopsy are known for diagnosis of tumors.

Somatic genomic testing has become the standard of care in a variety of cancers for diagnostic refinement, risk stratification, and therapeutic approach.

These methods can be categorized in different ways. Based on the techniques, there are polymerase chain reaction (PCR)- or next generation sequencing (NGS)-based sequencing. PCR-based sequencing can be used for single-locus/multiplexed assays and targeted panel, while NGS-based sequencing can be applied to any panel size, including genome-wide sequencing (4,5). In recent years, the rapid development of NGS technologies has led to a significant reduction in sequencing cost with improved accuracy and sensitivity. In the area of tumor biomarkers identification, NGS has been applied to sequence tumor DNA (tDNA) and circulating tumor DNA (ctDNA) in several kinds of cancer (4,5). In particular, the use of this methodology to develop a non-invasive diagnostic tool is the main goal of several scientists.

Tests based on changes of DNA in liquid biopsies have emerged as a promising molecular tool for cancer management. Indeed, during tumor development, tumor cells release their nucleic acids into the saliva and blood circulation. As circulating cell-free tumor nucleic acids remain stable in bodily fluids and may reflect the characteristics of tumor and/or premalignant lesions, they may be excellent non-invasive biomarkers in assessing cancer progression for patients whose tissue is not available (6-7).

Recently, mutational profile of some genes, including TP53, from circulating tumor DNA has been detected in HNSCC patients, but its implication in early diagnosis of local recurrence or minimal residual disease has not yet been clearly demonstrated (8; 11).

In particular, two are the most relevant works studying ctDNAs in liquid biopsy of HNSCC patients (8; 11). In Wang et al, 2015 the authors analyzed matched tissues, blood and saliva samples from 93 HNSCC patients by Safe-SeqS method, which is a sensitive PCR-based digital approach for the detection of low-frequency mutations based on massively parallel sequencing. By this technology able to detect rare mutations, the authors identified circulating mutations in a range of 70-100% of patients for four locus (TP53, PIK3CA, NOTCH1, and CDKN2A). This variation is linked to the kind of biological sample (saliva or plasma) and the tumor site analyzed: saliva is preferentially enriched for tumor DNA from oral cavity, whereas plasma is preferentially enriched for tumor DNA for other sites (pharynx and larynx). On the contrary, Perdomo et al show that the concordance in mutation detection between tumor and liquid biopsy is very low (>10%). In this study, the authors analyzed mutational profile of TP53, NOTCH1, CDKN2A, CASP8, PTEN genes from ctDNA of saliva and plasma samples, by target sequencing and NGS methods using two different HNSCC cohorts.

In recent years, assays for single-target detection have been replaced by next-generation sequencing (NGS) or massively parallel sequencing. Enabling high-throughput molecular analysis NGS allows for the simultaneous evaluation of many genes and the generation of millions of short nucleic acid sequences in parallel. The NGS high-throughput platform is more efficient and less expensive and provides information that is not provided by single gene-by-gene Sanger DNA sequencing analysis or by gene-specific targeted hot spot mutation assays. The vast number of variants identified by NGS in tumor tissue is attributed to the complexity of carcinogenesis, including the multistep process of genetic mutations and tumor heterogeneity (i.e., multiple clones of cells with related but distinct molecular signatures within tumors). Moreover, as a tumor progresses, it continues to acquire additional alterations that can affect the response to therapeutic agents such as chemotherapy or targeted therapies.

The targeted panel sequencing of selected cancer-related genes and genomic regions has been widely adopted in routine molecular diagnostics for common tumor, such as colon, lung and breast cancer, and is offered by several companies. Commercially available panels have been designed to detect mutation in tissue or in plasma only and they are based on hotspot mutations (specific mutations identified by previous studies and highly expressed). An example of commercially available panel is the Oncomine^{™} Precision Assay on the Ion Torrent^{™} chip, which detects, on tissue samples, hotspot somatic mutations in 50 cancer-related genes and it is used for different solid tumors. WO-2017/077499 relates to a method of detecting head and neck squamous cell carcinoma (HNSCC) in a sample having or suspected of having the HNSCC, said method comprising a step of detecting aberration in TP53, CASP8, OBSCN, ING1, TTK, U2AF1, RASA1, CDKN2A, NOTCH1, NOTCH2, DMD, PIK3CA, AJUBA, ANK3, FAT1, HLAA, KEAP1, KMT2D and NFE212.

However, although NGS-based cancer panel tests have been offered by several companies and widely adopted in routine molecular diagnostics for common tumors, no methodology for mutational profiling nor specific mutational target panels for rare tumors such as H&N tumors are currently available.

In addition, none of the currently available panels is capable to perform the simultaneous assessment of tissue and plasma.

According to the present invention, it has now been surprisingly found that detecting mutations only in three specific head and neck cancer-related genes provides a diagnostic method that is characterized by very high sensibility and specificity. In particular, the present invention provides a new method for in vitro diagnosis of head and neck cancer based on detecting mutations in the following three genes: TP53, CDKN2A and FAT1 genes.

The method of the invention can be advantageously used for detecting, monitoring and predicting the course of head and neck squamous cell carcinoma (HNSCC) with high specificity and sensibility.

The sequencing of the above-mentioned genes according to the present invention allows identifying known and unknown mutations of the above-mentioned genes.

For example, the method according to the invention can be carried out by an NGS-based specific mutational panel (hereinafter also referred to as H&N chip) containing the entire coding DNA sequence of TP53, CDKN2A and FAT1 genes.

According to the method of the present invention, it is possible to test both tissue and liquid samples, such as plasma or saliva. The vast majority of the HNSCC contain somatic mutations that are specific to the cancer. As a consequence, by testing both tissue and plasma of a subject, an extremely high level of patients affected by HNSCC can be identified.

Known approaches for identifying tumors are based on the detection of only specific mutations with high resolution (i.e. PCR based methodology) or multiple mutations located in different genes with low resolution(NGS-based methodology). In addition, the available mutational panels developed for identifying mutations in several genes cancer-related, don't include all the coding regions of all genes. For example, the Oncomine panel includes only the hotspot mutations for all genes except for TP53. The method according to the present invention, based on NGS technology, uses a new panel of genes that overcomes the limits of known methods by providing investigation of a wide range of mutations with high resolutions. Of note, this panel has been developed to analyze with high sensitivity, all the coding regions of all included genes. Accuracy of the method according to the present invention is very high, with a sensibility and specificity >90% as calculated using as reference dPCR-based method (considered the gold standard methodology for single mutation detection with a resolution for allele frequency detection > 0.1%).

It is known that head and neck cancers are extremely heterogeneous, this means that the allelic frequency levels are very low (<1%). The method according to the present invention is able to detect mutations with very low allelic frequencies (<1%) and to identify a large population of patients with H&NSpecifically, through the assay of the entire coding sequence of TP53, CDKN2A and FAT1, somatic mutations with an allele frequency >0.2% may be detected in plasma or tissue samples (fresh, frozen and formalin-fixed paraffin-embedded (FFPE)), including lymph nodes. Those somatic mutations may be used in subsequent assays in the same or different sample types. Samples may be collected at multiple time points and assayed in real time, close to the time of collection, or they may be held and assayed in batches.

The experimental data reported below describe the mutational profile analysis of 89 HNSCC cases and show that tumor-derived DNA can be detected in the plasma or/and tissue by the method and kit of the present invention with high sensitivity and sensibility. Tumor DNA was detectable in every one of the 89 patients with cancers and the fraction of mutant DNA in the samples was similar to those observed with dPCR.

Therefore, the method and kit according to the present invention present the following characteristics and advantages.

The method and kit of the invention are capable of identifying HNSCC patients with high specificity, since they are based on the entire CDS of the specific genes TP53, CDKN2A and FAT1, so that they allow the detection of known and unknown mutations present in the patient samples. Indeed, although the number of genes is significantly lower than other available custom cancer panels, the experimental data reported below show that more than 90% of HNSCC patients are identified by the method and kit of the present invention, whereas only 80% of HNSCC patients was recognized by the commercially available Oncomine Tissue^{™} panel, even though the latter includes a higher number of genes (50 genes). No other panels exists that uses the entire coding region of only these three genes (TP53, CDKN2A and FAT1) in the mutational profile.

The method and kit according to the present invention provide high accuracy of the assay; the presence of only three genes confers to the panel higher sensibility compared to commercially available panels. This high accuracy is due to the exquisite balancing between coverage and number of genes obtained with this panel.

The method and kit according to the present invention are advantageously capable of detecting the presence of mutations also when present in a small fraction of cells.

In addition, when applied in tumor samples, the method and kit according to the present invention allow characterizing tumor heterogeneity.

The method and kit of the invention are able to identify the presence of tumor cells in lymph node samples and recognize positive lymph nodes.

In addition, the method and kit of the invention are able to detect circulating DNA released by tumor cells in plasma samples and reveal potential tumor cells spreading. The method according to the invention is the first one developed and available for simultaneous assessment of tissue and plasma of cancer patients.

The method and kit of the present invention can also be advantageously applied in the diagnosis of residual tumor cells in the resection margins; therefore, they are able to support surgical strategy management of HNSCC by decreasing the risk of developing recurrence or a second tumor also in patients whose resection margins appear tumor-free by histology.

In addition, the method and kit of the present invention can be advantageously applied in early detection of tumor, disease monitoring and surveillance. In particular, the possibility to examine saliva or mucosal samples by the method of the present invention allows early detection of mutations. Similarly, for monitoring the disease progression after curative treatment, the method according to the present invention is able to detect residual cancer cells from tissue sample of the resection margin, while for possible recurrences saliva and/or plasma samples can be used. The experimental data reported below show that tumor DNA in the plasma and/or resection margin has been detected in patients developing recurrence only months later.

Therefore, the role of the mutational analysis by H&N chip according to the present invention can be different by using different biological samples. In fact, the analysis of tumor tissues can help the diagnosis of HNSCC patients and the choice of treatment strategy, while the analysis of the resection margins coupled with the histology can help the clinicians to diagnose the minimal residual disease or to predict a high probability to develop recurrence for that patient. The analysis of histological negative lymph nodes can help the diagnosis of micro metastasis.

Finally, the analysis of the liquid biopsy samples during the follow-up of patients can help the clinicians to monitor the therapy response, to detect early recurrence and to choose an alternative therapy if the current one doesn't work.

Thus, the introduction of the method and kit according to the present invention in the clinical management of HNSCC patients will greatly improve the diagnostic accuracy of visual screening, as their detection in bodily fluids will serve as a warning message for disease development. Most importantly, early detection of HNSCC during surveillance will improve survival rates. Subsequently, earlier intervention would decrease the burden of disease. As early diagnosis of HNSCC would significantly improve patients' long-term survival and quality of life, the approach proposed by the method of the present invention has the potential to save several thousands of lives worldwide each year. Besides the enormous impact on patients' health, this will also have a positive impact on the economy, as much less funds will be needed for treatment and more people will be cured earlier and subsequently return to normal life and work.

Because of the current lack of an effective diagnostic system for early diagnosis and relapses or minimal residual disease in the HNSCC, the introduction of a H&N chip according to the method of the present invention as methodology in the HNSCC management could be extremely interesting for identify a suspicion of precancerous lesions or relapse at a very early stage and direct towards more invasive, costly and instrumentation in saturation diagnostic studies and laboratories, where only cases with positive diagnosis of biomarkers will be given priority.

Therefore, the new method according to the present invention will contribute to make the diagnostic process more fluid and at the same time more effective and less costly, further reducing stress and costs for patients and health costs, both for the public and private systems.

It is therefore a specific object of the present invention a method for the *in vitro* diagnosis of head and neck cancer, said method comprising detecting, in a biological sample, mutations in genes consisting of TP53, CDKN2A and FAT1 genes, wherein the presence of at least one mutation in one, two or all of said genes in comparison to the respective wild type sequence indicates the presence of head and neck cancer, wherein the method does not comprise the detection of mutations or other aberrations in other genes different from TP53, CDKN2A and FAT1 genes, apart from the optional detection of possible one or more specific mutations in PIK3CA gene. Therefore, the method comprises the detection of mutations only in TP53, CDKN2A and FAT1 genes or in these genes and in specific parts of PIK3CA gene. The specific mutations (hotspots) to be detected in PIK3CA gene are listed in table 1 further below.

As demonstrated in tables 5 and 7, although the number of the included genes in the chip of the invention is low, it is able to detect mutations with a higher frequency than other mutational panels including more genes.

In addition, according to the method of the present invention, the presence of a total of at least two mutations in said one, two or all of said genes in comparison to the respective wild type sequence can advantageously indicate the presence of head and neck cancer which is a immunotherapy responsive head and neck cancer and/or head and neck cancer in advanced stage, wherein said at least two mutations are present in the same gene or in different genes among said one, two or all of said genes.

According to the method of the present invention, the step of detecting mutations can be carried out on the entire coding regions and part of intronic region (in order to include the entire exons) of TP53, CDKN2A and FAT1 genes.

The method of the present invention can be applied on a biological sample chosen from the group consisting of a liquid biological sample, such as a liquid biopsy, for example plasma sample or saliva sample, or a tissue sample, such as a tumor sample, lymph node sample or a resection margin sample, wherein said biological sample is fresh, frozen or formalin-fixed paraffin-embedded.

The method of the present invention is advantageously able to identify head and neck cancer cells or circulating tumour DNA. According to an embodiment of the present invention, the method can be carried out on both a liquid sample and a tissue sample. The use of different samples provides a higher sensibility.

In particular, the method of the present invention can comprise or consist of the following steps:
a) extracting DNA from a biological sample;
b) sequencing all of TP53, CDKN2A and FAT1 genes; and
c) detecting mutations in said genes by comparing each sequence of said genes obtained in step b) with the respective wild type sequence.

In particular, the step of sequencing said all of TP53, CDKN2A and FAT1 genes is carried out by primers for the amplification of said genes, wherein each primer pair has respectively initial position of forward primer and final position of reverse primer as reported in table 2. According to the present invention, the method does not comprise the detection of a mutation or other aberrations in other genes different from TP53, CDKN2A and FAT1 genes, apart from the optional detection of specific mutations in PIK3CA gene. Therefore, the method comprises the detection of mutations only in TP53, CDKN2A and FAT1 genes or in these genes and in specific parts of PIK3CA gene. The specific mutations of PIK3CA gene are listed in table 1 further below. According to the method of the present invention, the step of detecting mutations in TP53, CDKN2A and FAT1 genes is carried out by sequencing the entire coding regions and part of intronic region (in order to include the entire exons) of TP53, CDKN2A and FAT1 genes.

**Table 1**

| **Gene** | **Accession_number** | **COSMIC_i d** | **CDS_mut_sy ntax** | **AA_mut_synt ax** | **HG19_coordina tes** |
|---|---|---|---|---|---|
| **PIK3CA** | NM_006218.1 | 12458 | c.1634A>C | p.E545A | 3:178936092-178936092 |
| **PIK3CA** | NM_006218.1 | 12459 | c.1637A>G | p.Q546R | 3:178936095-178936095 |
| **PIK3CA** | NM_006218.1 | 12461 | c.3062A>G | p.Y1021C | 3:178952007-178952007 |
| **PIK3CA** | NM_006218.1 | 12463 | c.3128T>C | p. M 1043T | 3:178952073-178952073 |
| **PIK3CA** | NM_006218.1 | 12464 | c.3204_3205 insA | p. N 1068fs*4 | 3:178952149-178952150 |
| **PIK3CA** | NM_006218.1 | 12580 | c.333G>C | p. K 111N | 3:178916946-178916946 |
| **PIK3CA** | NM_006218.1 | 12582 | c.1252G>A | p.E418K | 3:178927974-178927974 |
| **PIK3CA** | NM_006218.1 | 12584 | c.1357G>A | p.E453K | 3:178928079-178928079 |
| **PIK3CA** | NM_006218.1 | 12590 | c.3073A>T | p.T1025S | 3:178952018-178952018 |
| **PIK3CA** | NM_006218.1 | 12591 | c.3127A>G | p. M 1043V | 3:178952072-178952072 |
| **PIK3CA** | NM_006218.1 | 12592 | c.3132T>A | p. N 1044 K | 3:178952077-178952077 |
| **PIK3CA** | NM_006218.1 | 12597 | c.3145G>C | P.G1049R | 3:178952090-178952090 |
| **PIK3CA** | NM_006218.1 | 13570 | c.331A>G | p.K111E | 3:178916944-178916944 |
| **PIK3CA** | NM_006218.1 | 13594 | c.3068G>A | p.R1023Q | 3:178952013-178952013 |
| **PIK3CA** | NM_006218.1 | 17442 | c.1624G>C | p.E542Q | 3:178936082-178936082 |
| **PIK3CA** | NM_006218.1 | 17444 | c.3061T>C | p.Y1021H | 3:178952006-178952006 |
| **PIK3CA** | NM_006218.1 | 17445 | c.3104C>T | p.A1035V | 3:178952049-178952049 |
| **PIK3CA** | NM_006218.1 | 17449 | c.3207A>G | p.*1069_*106 9insWKDN* | 3:178952152-178952152 |
| **PIK3CA** | NM_006218.1 | 21451 | c.3075C>T | p.T1025T | 3:178952020-178952020 |
| **PIK3CA** | NM_006218.1 | 21462 | c.971C>T | p.T324I | 3:178921489-178921489 |
| **PIK3CA** | NM_006218.1 | 22540 | c.1031T>G | p.V344G | 3:178921549-178921549 |
| **PIK3CA** | NM_006218.1 | 24712 | c.1638G>T | p.Q546H | 3:178936096-178936096 |
| **PIK3CA** | NM_006218.1 | 24714 | c.3141T>G | p.H1047Q | 3:178952086-178952086 |
| **PIK3CA** | NM_006218.1 | 249872 | c.1631C>A | p.T544N | 3:178936089-178936089 |
| **PIK3CA** | NM_006218.1 | 249908 | c.3207+29T> C | p.? | 3:178952181-178952181 |
| **PIK3CA** | NM_006218.1 | 25041 | c.1637A>T | p.Q546L | 3:178936095-178936095 |
| **PIK3CA** | NM_006218.1 | 25085 | c.3120G>A | p. M 10401 | 3:178952065-178952065 |
| **PIK3CA** | NM_006218.1 | 25086 | c.3133G>A | p.D1045N | 3:178952078-178952078 |
| **PIK3CA** | NM_006218.1 | 27133 | c.1633G>C | p.E545Q | 3:178936091-178936091 |
| **PIK3CA** | NM_006218.1 | 27134 | c.3130A>G | p. N 1044 D | 3:178952075-178952075 |
| **PIK3CA** | NM_006218.1 | 27155 | c.1634A>T | p.E545V | 3:178936092-178936092 |
| **PIK3CA** | NM_006218.1 | 27156 | c.3137C>A | p.A1046E | 3:178952082-178952082 |
| **PIK3CA** | NM_006218.1 | 27158 | c.3146G>C | P.G1049A | 3:178952091-178952091 |
| **PIK3CA** | NM_006218.1 | 27273 | c.3136G>A | p.A1046T | 3:178952081-178952081 |
| **PIK3CA** | NM_006218.1 | 27374 | c.1635G>C | p.E545D | 3:178936093-178936093 |
| **PIK3CA** | NM_006218.1 | 27502 | c.241G>A | p.E81K | 3:178916854-178916854 |
| **PIK3CA** | NM_006218.1 | 27505 | c.333G>T | p.K111N | 3:178916946-178916946 |
| **PIK3CA** | NM_006218.1 | 28938 | c.3059C>T | p.A1020V | 3:178952004-178952004 |
| **PIK3CA** | NM_006218.1 | 29110 | c.3115T>C | p.F1039L | 3:178952060-178952060 |
| **PIK3CA** | NM_006218.1 | 29313 | c.3129G>A | p.M1043I | 3:178952074-178952074 |
| **PIK3CA** | NM_006218.1 | 36285 | c.3074C>T | p.T1025I | 3:178952019-178952019 |
| **PIK3CA** | NM_006218.1 | 36286 | c.3085G>C | p.D1029H | 3:178952030-178952030 |
| **PIK3CA** | NM_006218.1 | 36289 | c.3143A>G | p.H1048R | 3:178952088-178952088 |
| **PIK3CA** | NM_006218.1 | 6147 | c.1636C>G | p.Q546E | 3:178936094-178936094 |
| **PIK3CA** | NM_006218.1 | 746 | c.263G>A | p.R88Q | 3:178916876-178916876 |
| **PIK3CA** | NM_006218.1 | 754 | c.1035T>A | p.N345K | 3:178921553-178921553 |
| **PIK3CA** | NM_006218.1 | 757 | c.1258T>C | p.C420R | 3:178927980-178927980 |
| **PIK3CA** | NM_006218.1 | 759 | c.1616C>G | p.P539R | 3:178936074-178936074 |
| **PIK3CA** | NM_006218.1 | 760 | c.1624G>A | p.E542K | 3:178936082-178936082 |
| **PIK3CA** | NM_006218.1 | 762 | c.1625A>T | p.E542V | 3:178936083-178936083 |
| **PIK3CA** | NM_006218.1 | 763 | c.1633G>A | P.E545K | 3:178936091-178936091 |
| **PIK3CA** | NM_006218.1 | 764 | c.1634A>G | p.E545G | 3:178936092-178936092 |
| **PIK3CA** | NM_006218.1 | 765 | c.1635G>T | p.E545D | 3:178936093-178936093 |
| **PIK3CA** | NM_006218.1 | 766 | c.1636C>A | p.Q546K | 3:178936094-178936094 |
| **PIK3CA** | NM_006218.1 | 767 | c.1637A>C | p.Q546P | 3:178936095-178936095 |
| **PIK3CA** | NM_006218.1 | 769 | c.2702G>T | p.C901F | 3:178947827-178947827 |
| **PIK3CA** | NM_006218.1 | 770 | c.2725T>C | p.F909L | 3:178947850-178947850 |
| **PIK3CA** | NM_006218.1 | 771 | c.3073A>G | p.T1025A | 3:178952018-178952018 |
| **PIK3CA** | NM_006218.1 | 772 | c.3074C>A | p.T1025N | 3:178952019-178952019 |
| **PIK3CA** | NM_006218.1 | 773 | c.3129G>T | p.M1043I | 3:178952074-178952074 |
| **PIK3CA** | NM_006218.1 | 774 | c.3139C>T | p. H 1047Y | 3:178952084-178952084 |
| **PIK3CA** | NM_006218.1 | 775 | c.3140A>G | p.H1047R | 3:178952085-178952085 |
| **PIK3CA** | NM_006218.1 | 776 | c.3140A>T | p.H1047L | 3:178952085-178952085 |
| **PIK3CA** | NM_006218.1 | 777 | c.3145G>A | P.G1049S | 3:178952090-178952090 |
| **PIK3CA** | NM_006218.1 | 778 | c.2102A>C | p.H701P | 3:178938860-178938860 |
| **PIK3CA** | NM_006218.1 | 94984 | c.3129G>C | p.M1043I | 3:178952074-178952074 |
| **PIK3CA** | NM_006218.1 | 125368 | c.344G>T | P.R115L | 3:178916957-178916957 |
| **PIK3CA** | NM_006218.1 | 12579 | c.178C>A | p.Q60K | 3:178916791-178916791 |
| **PIK3CA** | NM_006218.1 | 12589 | c.2727C>G | p.F909L | 3:178947852-178947852 |
| **PIK3CA** | NM_006218.1 | 14052 | c.332A>G | p.K111R | 3:178916945-178916945 |
| **PIK3CA** | NM_006218.1 | 163484 | c.238G>A | p.E80K | 3:178916851-178916851 |
| **PIK3CA** | NM_006218.1 | 166154 | c.337_342de ICTCAAT | p. L113_N 114d elLN | 3:178916950-178916955 |
| **PIK3CA** | NM_006218.1 | 21448 | c.337_339de ICTC | p.L113del | 3:178916950-178916952 |
| **PIK3CA** | NM_006218.1 | 230020 | c.1396C>T | P.P466S | 3:178928118-178928118 |
| **PIK3CA** | NM_006218.1 | 24710 | c.325_327de IGAA | p.E109del | 3:178916938-178916940 |
| **PIK3CA** | NM_006218.1 | 24711 | c.335T>A | p. I 112 N | 3:178916948-178916948 |
| **PIK3CA** | NM_006218.1 | 27490 | c.333_335de IGAT | p. K111_I 112> N | 3:178916946-178916948 |
| **PIK3CA** | NM_006218.1 | 27497 | c.323G>A | p.R108H | 3:178916936-178916936 |
| **PIK3CA** | NM_006218.1 | 27499 | c.321_323de ICCG | p.R108del | 3:178916934-178916936 |
| **PIK3CA** | NM_006218.1 | 39166 | c.223C>G | p.Q75E | 3:178916836-178916836 |
| **PIK3CA** | NM_006218.1 | 41785 | c.1371C>G | p.N457K | 3:178928093-178928093 |
| **PIK3CA** | NM_006218.1 | 51258 | c.1221C>G | p.C407W | 3:178927458-178927458 |
| **PIK3CA** | NM_006218.1 | 51259 | c.341_342in sCCTCAA | p.N114_R115i nsLN | 3:178916954-178916955 |
| **PIK3CA** | NM_006218.1 | 6145 | c.328G>A | p.E110K | 3:178916941-178916941 |
| **PIK3CA** | NM_006218.1 | 6149 | c.1214C>T | p.S405F | 3:178927451-178927451 |
| **PIK3CA** | NM_006218.1 | 6150 | c.1352_1366 del15 | p.G451_L456> V | 3:178928074-178928088 |
| **PIK3CA** | NM_006218.1 | 749 | c.323G>C | P.R108P | 3:178916936-178916936 |
| **PIK3CA** | NM_006218.1 | 750 | c.332_334de IAGA | p.K111del | 3:178916945-178916947 |
| **PIK3CA** | NM_006218.1 | 758 | c.1357G>C | p.E453Q | 3:178928079-178928079 |
| **PIK3CA** | NM_006218.1 | 86045 | c.1358A>C | p.E453A | 3:178928080-178928080 |
| **PIK3CA** | NM_006218.1 | 86949 | c.1404+10T> G | p.? | 3:178928136-178928136 |
| **PIK3CA** | NM_006218.1 | 86950 | c.1404+2_14 04+3insT | p.? | 3:178928128-178928129 |
| **PIK3CA** | NM_006218.1 | 87210 | c.1252-14C>A | p.? | 3:178927960-178927960 |
| **PIK3CA_ ENST000 0026396 7** | ENST00000263967 | 125370 | c.1633G>A | P.E545K | 3:178936091-178936091 |
| **PIK3CA_ ENST000 0026396 7** | ENST00000263967 | 94985 | c.3129G>C | p.M1043I | 3:178952074-178952074 |
| **PIK3CA_ ENST000 0026396** 7 | ENST00000263967 | 94986 | c.3140A>G | p.H1047R | 3:178952085-178952085 |
| **PIK3CA_ ENST000 0026396** 7 | ENST00000263967 | 94987 | c.3140A>T | p.H1047L | 3:178952085-178952085 |
| **PIK3CA** | | COSM8730 6 | | P.E726K REF=G | |
| **PIK3CA** | | | c.1645G>A | D549N | |

The method of the present invention can be carried out by Next Generation Sequencing technologies.

As shown below, it has been found that the method of the invention is able to detect mutations in TP53, CDKN2A and FAT1 genes with an allelic frequency > 0.2%).

As mentioned above the method according to the present invention can further comprises sequencing specific parts of PIK3CA gene in order to detect the following mutations in PIK3CA gene, said mutations having the following COSMIC_id numbers:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
769
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306.

The present invention concerns also a kit for the *in vitro* diagnosis of head and neck cancer, said kit comprising reagents for sequencing only TP53, CDKN2A and FAT1 genes and, optionally, also PIK3CA gene, whereas the kit does not comprise any reagent for sequencing other genes.

In particular, said suitable reagents can comprise primers for the amplification of genes consisting of TP53, CDKN2A and FAT1 genes, wherein for the amplification of said genes, each primer pair has respectively initial position of forward primer and final position of reverse primer as reported in table 2 or claim 13.

The kit can further comprise primers for detecting one or more of the following mutations in PIK3CA gene, said mutations having the following COSMIC_id numbers:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
769
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306.

The kit of the present invention can comprise or consists of a chip suitable for carrying out the method of the invention. In particular, the chip comprises the primers for the amplification of said TP53, CDKN2A and FAT1 genes, wherein each primer pair has respectively initial position of forward primer and final position of reverse primer as reported in table 2. According to the present invention, the chip does not comprise primers for the amplification of other genes different from TP53, CDKN2A and FAT1 genes, apart from optional primers for detecting specific mutations of PIK3CA gene listed in table 1, i.e for the optional amplification of specific parts of PIK3CA gene. Therefore, the chip according to the present invention is able to detect mutations only in TP53, CDKN2A and FAT1 genes or in these genes and in specific parts of PIK3CA gene wherein the specific mutations listed in table 1 can be present.

Molecular information useful for the planning of therapy can be obtained by the analysis of biological samples from HNSCC patients using the H&N chip of the present invention. In particular, several clinical trials of phase III and IV targeting PI3K pathway are currently ongoing in HNSCC tumors. The presence of mutation in PIK3CA gene in the analyzed HNSCC tissues will indicate that these patients may benefit of PI3K inhibitors treatment. In addition, even if currently there aren't ongoing clinical trials on TP53 gene specifically in HNSCC, it has been demonstrated that HNSCC patients carrying TP53 mutations may also benefit to PI3K inhibitors treatment also in combination with radiotherapy.

Also disclosed, but not part of the invention, is a method of treating HNSCC in a subject, the method comprising detecting, in a biological sample, at least one mutation in genes consisting of TP53, CDKN2A and FAT1 genes in comparison to the respective wild type sequence, and, when said at least one mutation is present, treating the subject with a suitable therapy against head and neck cancer, such as by administering an anti HNSCC drug, such as inhibitors of PI3K/Akt/mTOR Pathways and/or radiotherapy. Optionally, the method of treating HNSCC according to the present invention can comprise also detecting the specific mutations of PIK3CA gene listed in table 1.

In particular, when a total of at least two mutations in said one, two or all of said TP53, CDKN2A and FAT1 genes in comparison to the respective wild type sequence are detected, the method of treating HNSCC according to the present invention comprises or consists of administering a immunotherapy to the subject presenting said mutations.

The present invention now will be described by an illustrative, but not limitative way, according to preferred embodiments thereof, with particular reference to the examples.

### EXAMPLE 1: Study concerning the specificity and sensibility of the method for detecting head and neck cancer according to the present invention

### Material and methods

*The biological material of human origin used in these experiments has been sampled with the express, free and informed consent, for that sampling and utilization, of the person from whom the material was taken, based on applicable legislation.*

### IRE cohort

This is a collection of matched tumor, peritumor (taken at a distance greater than 1 cm from the tumor) and normal (surgical resection margin) samples from patients with histologically confirmed primary HNSCC undergoing curative treatment at the Otolaryngology Head and Neck Surgery Department of Regina Elena Cancer Institute (IRE, Rome, Italy). Matched plasma samples has been also collected before/ after surgery therapy and during follow-up for each patients. Only patients with primary tumor site from oral cavity, pharynx (oropharynx and ipopharynx) and larynx, histologically proven Squamous Cell Carcinoma (SCC) and who did not receive any anticancer therapy before surgery are included in the study. For each patient the information about the smoking history, alcohol use, HPV status (28 genotypes evaluated), TNM staging, disease relapse, metastasis formation, therapeutic regimen after surgery and follow-up were available. As a consequence of these inclusion/exclusion criteria, this cohort is mainly represented from HPV-negative. Recently, it has been included collection of saliva samples before and after surgery according to the Wang et al protocol (7).

### Study design

The strategy used to realize the panel was divided in two steps. The first consisted in the selection of the genes included in the H&N chip of the invention. The second step was a large preclinical validation study to determine the accuracy of the H&N chip protocol. The validation study involved a double blinded parallel evaluation, with both NGS-based H&N panel and dPCR techniques, of tumoral (t)DNA samples and cell free (cf)DNA from tissue and plasma, respectively. Consistency of NGS-based H&N panel mutational detection was assessed matching the results obtained with dPCR-based diagnoses, at the level of type and frequency of mutation detected.

### Selection of Genes

The selection of the genes has been done taking in consideration multiple scientific and technical aspects: the biological role of genes, their mutations frequency in HNSCC tumors, their structural information (size of the gene, number of mutations to be analyzed) and the technological characteristics of the analytical instruments.

In particular, a comprehensive reviews of the literature and genetic database were performed, including COSMIC (http://cancer.sanger.ac.uk/cosmic, last accessed January 20, 2019) and My Cancer Genome (http://www.mycancergenoma.org, last accessed January 20, 2019), to identify genes with the highest incidence of mutations in head and neck cancer according to this criteria, five genes have been selected: TP53, CDKN2A, FAT1, PIK3CA and NOTCH1. At a second step, tumor suppressor genes displayed prevalently by HPV negative patients were selected. Finally, only genes for which no ongoing clinical trials in HNSCC are currently under evaluation, has been included in the selection. To obtain a comprehensive panel including all possible mutation related to the selected genes, it was decided to include the entire coding sequence of the genes. Finally, the dimension of the panel and the number of included genes was chosen to obtain the highest coverage. By this multiple steps selection the following genes are included:
1. TP53, the most frequently mutated gene (frequency reported by TGCA and COSMIC: 41% to 71%). The mutation on this gene is associated with tumor resistance to radiotherapy and chemotherapy in HNSCC patients, which makes the TP53 mutation status a potentially useful molecular factor for risk stratification and predictor of clinical response in these patients;
2. FAT-1 (frequency reported by TGCA and COSMIC: 23-5%) one of the most frequent in H&N. FAT1 suppressed the migration and invasion capability of HNSCC cells. Functional analysis suggested that nonsense mutations in FAT1 result in the loss of the suppression of tumor progression. FAT1 mutations and downregulation defined nodal involvement, lymphovascular permeation and tumor recurrence. In addition, the clinical implications of FAT1 mutations in all entire coding region is poorly characterized and by this chip it is possible to identify if there are (and eventually what are) FAT1 mutations in specific codons more clinically relevant versus other ones.
3. CDKN2A (frequency reported by TGCA and COSMIC: 22-16%). It is one of the most frequent in H&N. It acts as a tumor suppressor by cell cycle control and its inactivation is involved in carcinogenesis, progression and metastasis of HNSCC. As FAT-1, it has the lowest number of mutations already noted (so that new ones can be identified).

While the following genes were excluded:
NOTCH1 and PIK3CA: Indeed, although both of them have a frequency (10-18% for NOTCH1 and 8-21% for PIK3CA)) similar to CDKN2A and FAT1, the study was focused on genes for which target therapies are not yet available, whereas pharmacologically targeting genes as NOTCH1 and PIK3CA for which ongoing clinical trials in HNSCC are largely under evaluation were excluded. In addition, PIK3CA and NOTCH1 were also excluded for technical reason: the length of these genes was too high to be included in the chip.

### Performance Validation and LOD analysis

The Seraseq^{®} ctDNA Mutation Mix v2 AF at 5%, 1%, 0.25%, 0.1% and 0% allelic frequencies mimicking human fragmented circulating free DNA (cfDNA) (average 170 bp) were used to assess Head &Neck panel performance validation and to define limit of detection (LOD). All artificial cfDNA samples were analysed in duplicate for a total of 50 mimic samples. In addition, 16 tumor DNA and 16 plasma cfDNA samples were used. On completion, 82 samples were acquired.

To assess the accuracy, sensitivity, and specificity of the approach, the number and frequency of the mutations detected with H&N panel were compared to those expected in the artificial cfDNA samples or diagnosed by dPCR approach. True positives (TPs) are those variants detected by the present pipeline and those expected in the mimic samples, whereas true negatives (TNs) are variants that were neither detected by the present pipeline nor expected in the artificial samples. Similarly, false positives (FPs) are variants detected by the present pipeline but were not expected in the mimic samples, whereas false negatives (FNs) are variants that were expected in the mimic samples but were not detected by the present pipeline. Accuracy was calculated as follows: (TP + TN)/(TP + FP + TN + FN). Sensitivity was calculated as follows: TP/(TP + FN). Specificity was calculated as follows: TN/(TN + FP).

To quantify the impact of variant allele frequency (VAF) on analytic sensitivity, variants were stratified by their VAF. In addition, sensitivity was assessed as a function of coverage when reads were down-sampled to simulate average coverage of 2000×, 6000×, 10000×, 14000×, and 25000×. Two reads that called the mutation and VAF >0.1% determined the cutoff for positive detection of SNVs in the bioinformatics pipeline.

### Samples collection and DNA extraction

The samples collection included FFPE and fresh frozen tissues with relative blood samples from untreated and full-annotated patients. In particular, fresh tissues included 89 tumor, 4 peritumor and 4 normal samples, awhile FFPE tissues included 9 tumor, 6 resection margins and 17 lymph nodes samples. Fresh tissues were collected at surgery and preserved in RNA later (Ambion), while blood sample has been collected in different times for each patient: before surgery, 24 hours, 15 days after surgery and during the follow-up. Blood (10ml) was drawn in BD Vacutainer K2EDTA tubes and processed within 1h. Plasma fraction has been prepared in one hour by centrifugation at 3500 rpm (1500 g) for 20 minutes at 4°C. Then it was stored at -80°C until using.

### DNA extraction

Sections (5 µm-thick) were cut from a representative formalin-fixed, paraffin-embedded (FFPE) tissue block. One section was counterstained by hematoxylin/eosin and assessed for quality and a tumor fraction ≥50% by an expert pathologist. The remaining sections were deparaffinized and digested overnight at 56°C with proteinase K (Qiagen, Hilden, Germany). DNA was extracted by the QlAmp DNA FFPE Tissue Kit (Qiagen) according to the manufacturer's instructions, and aliquoted in three different vials, each of which was given to three different users for testing in the two NGS platforms and dPCR extraction. No freeze-thawing cycles were allowed. Circulating free DNA (cfDNA) was extracted from 2-ml of plasma by the QIAmp circulating nucleic acid kit (Qiagen) according to the manufacturer's instructions in a final volume of 30 µL, and stored at -20 °C until NGS/dPCR analysis. Both tDNAs and cfDNA were fluorimetrically quantified with the Qubit dsDNA HS assay kit (Life Technologies, Carlsbad, CA, USA).

### DNA Template Preparation and Amplification

A multiplex PCR amplification strategy for the coding gene sequences was accomplished online (Ion AmpliseqTM Designer) to amplify the target region specified above with 25 base pair exon padding. After a comparison of several primer design options provided by a software Ion AmpliSeq Designer (Life Technologies, Carlsbad, CA, USA), the design providing the maximum target sequence coverage was chosen. The ordered 222 amplicons covered approximately 98.9% of the target sequence. Table 2 shows the chromosome in which gene is located and the chromosomal coordinates of the primers (forward and reverse) related to the investigated gene (hg19 human reference genome).

**Table 2**

| **Chromosome** | **initial position of forward primers** | **Final position of Reverse primers** | **GENE** |
|---|---|---|---|
| chr4 | 187509686 | 187509848 | FAT1 |
| chr4 | 187509780 | 187509957 | FAT1 |
| chr4 | 187509922 | 187510077 | FAT1 |
| chr4 | 187509985 | 187510157 | FAT1 |
| chr4 | 187510068 | 187510242 | FAT1 |
| chr4 | 187510198 | 187510363 | FAT1 |
| chr4 | 187510240 | 187510402 | FAT1 |
| chr4 | 187510358 | 187510528 | FAT1 |
| chr4 | 187516739 | 187516905 | FAT1 |
| chr4 | 187516839 | 187517007 | FAT1 |
| chr4 | 187516950 | 187517116 | FAT1 |
| chr4 | 187517558 | 187517725 | FAT1 |
| chr4 | 187517686 | 187517856 | FAT1 |
| chr4 | 187517722 | 187517895 | FAT1 |
| chr4 | 187517847 | 187518005 | FAT1 |
| chr4 | 187517899 | 187518066 | FAT1 |
| chr4 | 187518000 | 187518159 | FAT1 |
| chr4 | 187518082 | 187518255 | FAT1 |
| chr4 | 187518147 | 187518315 | FAT1 |
| chr4 | 187518290 | 187518451 | FAT1 |
| chr4 | 187518790 | 187518955 | FAT1 |
| chr4 | 187518844 | 187519010 | FAT1 |
| chr4 | 187519017 | 187519169 | FAT1 |
| chr4 | 187519125 | 187519285 | FAT1 |
| chr4 | 187519235 | 187519405 | FAT1 |
| chr4 | 187521006 | 187521177 | FAT1 |
| chr4 | 187521066 | 187521226 | FAT1 |
| chr4 | 187521171 | 187521342 | FAT1 |
| chr4 | 187521242 | 187521412 | FAT1 |
| chr4 | 187521376 | 187521545 | FAT1 |
| chr4 | 187521496 | 187521665 | FAT1 |
| chr4 | 187522275 | 187522435 | FAT1 |
| chr4 | 187522395 | 187522567 | FAT1 |
| chr4 | 187522457 | 187522627 | FAT1 |
| chr4 | 187523994 | 187524140 | FAT1 |
| chr4 | 187524081 | 187524248 | FAT1 |
| chr4 | 187524279 | 187524427 | FAT1 |
| chr4 | 187524386 | 187524526 | FAT1 |
| chr4 | 187524421 | 187524566 | FAT1 |
| chr4 | 187524522 | 187524666 | FAT1 |
| chr4 | 187524564 | 187524737 | FAT1 |
| chr4 | 187524660 | 187524834 | FAT1 |
| chr4 | 187524748 | 187524922 | FAT1 |
| chr4 | 187524831 | 187524973 | FAT1 |
| chr4 | 187524920 | 187525063 | FAT1 |
| chr4 | 187524986 | 187525139 | FAT1 |
| chr4 | 187525062 | 187525178 | FAT1 |
| chr4 | 187525498 | 187525664 | FAT1 |
| chr4 | 187525619 | 187525786 | FAT1 |
| chr4 | 187527123 | 187527289 | FAT1 |
| chr4 | 187527218 | 187527393 | FAT1 |
| chr4 | 187527324 | 187527496 | FAT1 |
| chr4 | 187530280 | 187530448 | FAT1 |
| chr4 | 187530335 | 187530508 | FAT1 |
| chr4 | 187530870 | 187531020 | FAT1 |
| chr4 | 187530976 | 187531145 | FAT1 |
| chr4 | 187531040 | 187531203 | FAT1 |
| chr4 | 187532465 | 187532639 | FAT1 |
| chr4 | 187532511 | 187532682 | FAT1 |
| chr4 | 187532635 | 187532805 | FAT1 |
| chr4 | 187532720 | 187532886 | FAT1 |
| chr4 | 187532798 | 187532940 | FAT1 |
| chr4 | 187534199 | 187534339 | FAT1 |
| chr4 | 187534304 | 187534429 | FAT1 |
| chr4 | 187534332 | 187534502 | FAT1 |
| chr4 | 187534438 | 187534606 | FAT1 |
| chr4 | 187535251 | 187535403 | FAT1 |
| chr4 | 187535344 | 187535503 | FAT1 |
| chr4 | 187535400 | 187535567 | FAT1 |
| chr4 | 187538056 | 187538192 | FAT1 |
| chr4 | 187538141 | 187538305 | FAT1 |
| chr4 | 187538188 | 187538352 | FAT1 |
| chr4 | 187538783 | 187538918 | FAT1 |
| chr4 | 187538869 | 187539025 | FAT1 |
| chr4 | 187538912 | 187539080 | FAT1 |
| chr4 | 187539017 | 187539189 | FAT1 |
| chr4 | 187539078 | 187539239 | FAT1 |
| chr4 | 187539190 | 187539350 | FAT1 |
| chr4 | 187539232 | 187539400 | FAT1 |
| chr4 | 187539355 | 187539504 | FAT1 |
| chr4 | 187539394 | 187539545 | FAT1 |
| chr4 | 187539501 | 187539622 | FAT1 |
| chr4 | 187539540 | 187539701 | FAT1 |
| chr4 | 187539627 | 187539764 | FAT1 |
| chr4 | 187539699 | 187539849 | FAT1 |
| chr4 | 187539759 | 187539920 | FAT1 |
| chr4 | 187539840 | 187540016 | FAT1 |
| chr4 | 187539911 | 187540065 | FAT1 |
| chr4 | 187540015 | 187540181 | FAT1 |
| chr4 | 187540073 | 187540223 | FAT1 |
| chr4 | 187540184 | 187540347 | FAT1 |
| chr4 | 187540236 | 187540403 | FAT1 |
| chr4 | 187540345 | 187540484 | FAT1 |
| chr4 | 187540400 | 187540524 | FAT1 |
| chr4 | 187540481 | 187540606 | FAT1 |
| chr4 | 187540538 | 187540679 | FAT1 |
| chr4 | 187540598 | 187540772 | FAT1 |
| chr4 | 187540687 | 187540831 | FAT1 |
| chr4 | 187540770 | 187540913 | FAT1 |
| chr4 | 187540830 | 187540956 | FAT1 |
| chr4 | 187540925 | 187541089 | FAT1 |
| chr4 | 187541016 | 187541184 | FAT1 |
| chr4 | 187541085 | 187541255 | FAT1 |
| chr4 | 187541183 | 187541352 | FAT1 |
| chr4 | 187541254 | 187541424 | FAT1 |
| chr4 | 187541348 | 187541504 | FAT1 |
| chr4 | 187541418 | 187541580 | FAT1 |
| chr4 | 187541507 | 187541669 | FAT1 |
| chr4 | 187541577 | 187541726 | FAT1 |
| chr4 | 187541666 | 187541809 | FAT1 |
| chr4 | 187541724 | 187541893 | FAT1 |
| chr4 | 187541813 | 187541942 | FAT1 |
| chr4 | 187541887 | 187542026 | FAT1 |
| chr4 | 187541943 | 187542110 | FAT1 |
| chr4 | 187542025 | 187542176 | FAT1 |
| chr4 | 187542120 | 187542276 | FAT1 |
| chr4 | 187542186 | 187542360 | FAT1 |
| chr4 | 187542279 | 187542400 | FAT1 |
| chr4 | 187542353 | 187542509 | FAT1 |
| chr4 | 187542400 | 187542562 | FAT1 |
| chr4 | 187542504 | 187542668 | FAT1 |
| chr4 | 187542558 | 187542717 | FAT1 |
| chr4 | 187542661 | 187542813 | FAT1 |
| chr4 | 187542760 | 187542906 | FAT1 |
| chr4 | 187542806 | 187542967 | FAT1 |
| chr4 | 187549198 | 187549361 | FAT1 |
| chr4 | 187549316 | 187549490 | FAT1 |
| chr4 | 187549385 | 187549556 | FAT1 |
| chr4 | 187549591 | 187549765 | FAT1 |
| chr4 | 187549720 | 187549886 | FAT1 |
| chr4 | 187549777 | 187549937 | FAT1 |
| chr4 | 187549890 | 187550054 | FAT1 |
| chr4 | 187554804 | 187554976 | FAT1 |
| chr4 | 187554897 | 187555061 | FAT1 |
| chr4 | 187557084 | 187557247 | FAT1 |
| chr4 | 187557176 | 187557344 | FAT1 |
| chr4 | 187557249 | 187557410 | FAT1 |
| chr4 | 187557365 | 187557537 | FAT1 |
| chr4 | 187557616 | 187557791 | FAT1 |
| chr4 | 187557747 | 187557889 | FAT1 |
| chr4 | 187557818 | 187557984 | FAT1 |
| chr4 | 187557903 | 187558071 | FAT1 |
| chr4 | 187557985 | 187558149 | FAT1 |
| chr4 | 187560789 | 187560948 | FAT1 |
| chr4 | 187560877 | 187561020 | FAT1 |
| chr4 | 187584406 | 187584550 | FAT1 |
| chr4 | 187584482 | 187584647 | FAT1 |
| chr4 | 187584544 | 187584715 | FAT1 |
| chr4 | 187584650 | 187584817 | FAT1 |
| chr4 | 187627676 | 187627834 | FAT1 |
| chr4 | 187627720 | 187627886 | FAT1 |
| chr4 | 187627828 | 187627971 | FAT1 |
| chr4 | 187627889 | 187628050 | FAT1 |
| chr4 | 187627968 | 187628133 | FAT1 |
| chr4 | 187628051 | 187628214 | FAT1 |
| chr4 | 187628126 | 187628278 | FAT1 |
| chr4 | 187628217 | 187628366 | FAT1 |
| chr4 | 187628288 | 187628462 | FAT1 |
| chr4 | 187628360 | 187628529 | FAT1 |
| chr4 | 187628459 | 187628623 | FAT1 |
| chr4 | 187628528 | 187628691 | FAT1 |
| chr4 | 187628623 | 187628792 | FAT1 |
| chr4 | 187628696 | 187628862 | FAT1 |
| chr4 | 187628784 | 187628927 | FAT1 |
| chr4 | 187628863 | 187628989 | FAT1 |
| chr4 | 187628920 | 187629077 | FAT1 |
| chr4 | 187629015 | 187629179 | FAT1 |
| chr4 | 187629069 | 187629237 | FAT1 |
| chr4 | 187629181 | 187629298 | FAT1 |
| chr4 | 187629229 | 187629396 | FAT1 |
| chr4 | 187629305 | 187629458 | FAT1 |
| chr4 | 187629390 | 187629561 | FAT1 |
| chr4 | 187629454 | 187629600 | FAT1 |
| chr4 | 187629553 | 187629694 | FAT1 |
| chr4 | 187629606 | 187629728 | FAT1 |
| chr4 | 187629686 | 187629836 | FAT1 |
| chr4 | 187629785 | 187629925 | FAT1 |
| chr4 | 187629830 | 187630003 | FAT1 |
| chr4 | 187629924 | 187630093 | FAT1 |
| chr4 | 187629997 | 187630171 | FAT1 |
| chr4 | 187630090 | 187630246 | FAT1 |
| chr4 | 187630182 | 187630351 | FAT1 |
| chr4 | 187630276 | 187630408 | FAT1 |
| chr4 | 187630355 | 187630518 | FAT1 |
| chr4 | 187630437 | 187630574 | FAT1 |
| chr4 | 187630516 | 187630682 | FAT1 |
| chr4 | 187630616 | 187630785 | FAT1 |
| chr4 | 187630687 | 187630853 | FAT1 |
| chr4 | 187630782 | 187630954 | FAT1 |
| chr4 | 187630858 | 187631029 | FAT1 |
| chr9 | 21968149 | 21968274 | CDKN2A |
| chr9 | 21968651 | 21968801 | CDKN2A |
| chr9 | 21970835 | 21971009 | CDKN2A |
| chr9 | 21970962 | 21971143 | CDKN2A |
| chr9 | 21971106 | 21971273 | CDKN2A |
| chr9 | 21974425 | 21974592 | CDKN2A |
| chr9 | 21974542 | 21974709 | CDKN2A |
| chr9 | 21974650 | 21974814 | CDKN2A |
| chr9 | 21994109 | 21994285 | CDKN2A |
| chr9 | 21994241 | 21994398 | CDKN2A |
| chr17 | 7572830 | 7573002 | TP53 |
| chr17 | 7572907 | 7573051 | TP53 |
| chr17 | 7573856 | 7574026 | TP53 |
| chr17 | 7573989 | 7574156 | TP53 |
| chr17 | 7576473 | 7576588 | TP53 |
| chr17 | 7576544 | 7576709 | TP53 |
| chr17 | 7576807 | 7576971 | TP53 |
| chr17 | 7576927 | 7577092 | TP53 |
| chr17 | 7577006 | 7577176 | TP53 |
| chr17 | 7577097 | 7577256 | TP53 |
| chr17 | 7577375 | 7577531 | TP53 |
| chr17 | 7577486 | 7577639 | TP53 |
| chr17 | 7578120 | 7578282 | TP53 |
| chr17 | 7578239 | 7578398 | TP53 |
| chr17 | 7578281 | 7578453 | TP53 |
| chr17 | 7578403 | 7578580 | TP53 |
| chr17 | 7579230 | 7579398 | TP53 |
| chr17 | 7579327 | 7579505 | TP53 |
| chr17 | 7579486 | 7579616 | TP53 |
| chr17 | 7579596 | 7579770 | TP53 |
| chr17 | 7579737 | 7579874 | TP53 |
| chr17 | 7579817 | 7579988 | TP53 |

A total of up to 10 ng DNA per sample was used for target amplification by a multiple PCR using the Ion AmpliSeq Library Kit Plus according to the manufacturer's procedure (no OGM are included in the kit).

After each pool had undergone 18 PCR cycles, the PCR primers were removed with FuPa Reagent and the amplicons were ligated to the sequencing adaptors with short stretches of index sequences (barcodes) that enabled sample multiplexing for subsequent steps (Ion XpressTM Barcode Adapters Kit; Life Technologies). After purification with AMPure XP beads (Beckman Coulter, Krefeld, Germany), the barcoded libraries were quantified with a Qubit^{®} 2.0 Fluorimeter (Life Technologies, Darmstadt, Germany) and pooled according to their concentration to obtain a final library pool of 75 pmol/l. To clonally amplify the library DNA onto the Ion Sphere Particles (ISPs; Life Technologies, Darmstadt, Germany), the library pool was subjected to emulsion PCR by using the Ion Chef Templating/Enrichment System following the manufacturer's protocol.

For the template preparation, up to 16 libraries were pooled together and loaded on Ion Chef Instrument; sequencing was subsequently performed using Ion chip 540^{™} (60 to 80 × 10⁶ of reads capability) on Ion S5 Instrument.

### Sequencing

The prepared libraries were then sequenced on an Ion S5 Sequencer using an Ion 540 Chip and an Ion 540 kit-Chef (all Thermo Fisher Scientific) as per the manufacturer's instructions with configuration500 flows covering the 200 bp library read length. Enriched ISPs which carried many copies of the same DNA fragment were subjected to sequencing on an Ion 540^{™} chip to sequence pooled libraries with up to 16 samples.

During this process, the incorporation of each nucleotide causes the release of hydrogen and pyrophosphate: the hydrogen ion that is released in the reaction changes the pH of the solution, which is detected by an ion-sensitive field-effect transistor (ISFET). This process commonly referred to as base-calling.

In this report, data from plasma and artificial cfDNA collected during the assay validation phase were used for developing QC metrics. The number of combined libraries that can be accommodated in a single sequencing run depends on the size of the chip, number of samples, and the coverage required for each sample.

### Data Analysis

### Bioinformatics Generation of Sequence Information

Generated raw sequence data in FASTQ format were aligned to the hg19 human reference genome using the Torrent Mapping Alignment Program aligner implemented in v5.2 of the Torrent Suite software (Thermo Fisher Scientific). For SNV calling, two software programs in parallel-plug-in Torrent Variant Caller were used with customized parameters for calling of low frequencies mutations (Thermo Fisher Scientific).

The Ion Reporter Workflow was customized in order to find low frequency variant, by setting the following parameters:
Data Quality Stringency: 7
Downsample To Coverage: 20000
Snp Min Cov Each Strand: 4
Snp Min Variant Score: 6
Snp Min Allele Freq: 0.005
Snp Min Coverage: 5
SNP Strand Bias: 0.95
Indel Min Cov Each Strand: 4
Indel Min Variant Score: 6
Indel Min Allele Freq: 0.02
Indel Min Coverage: 100
Indel Strand Bias: 0.9
Prediction Precision: 1.0
Outlier Probability: 0.005
Heavy Tailed: 3
Filter Unusual Predictions: 0.12
Filter Insertion Predictions: 0.2
Filter Deletion Predictions: 0.2
HP Max Length: 8
Mnp Min Cov Each Strand: 4
Mnp Min Variant Score: 6
Mnp Min Allele Freq: 0.02
Mnp Min Coverage: 10
MNP Strand Bias: 0.95
MNP Strand Bias Pval: 0.01
SNP Strand Bias Pval: 0.01
INDEL Strand Bias Pval: 1.0
Position Bias Reference Fraction: 0.05
Position Bias: 0.75
Position Bias Pvalue: 0.05
FD Nonsnp Min Var Cov: 1
Minimum mapping qv: 4
Read SNP Limit: 10
Read Max Mismatch Fraction: 1.0
Generate Min Alt Allele Freq: 0.01
Generate Min Indel Alt Allele Freq: 0.01
Generate Min Coverage: 6
Min Var Count: 5
Min Var Freq: 0.15
Relative Strand Bias: 0.8

Final analysis of the founded variant was performed by applying a filter chain to leave out common SNPs and homozygous variants.

### Assay Validation

### Droplet digital PCR (ddPCR)

Validation of *TP53* mutations for a subgroup of samples was performed by QX200 ddPCR^{™} System (Bio-Rad Laboratories, Inc., Hercules, CA, USA) using ddPCR Mutation Detection Assays (FAM-Mutation assay: dHsaCP2000019 and HEX-wild type assay: dHsaCP2000020).

### Direct Sequencing of TP53

Validation of TP53 mutations was also performed by direct Sequencing of TP53 mutation in a subgroup of 18 HNSCC tumor tissues by Genechron Biotech Company.

### Results

### Analytical validation of H&N chip

To test and validate the sensitivity of the chip, cell free DNA (cfDNA)samples (Seraseq^{®} ctDNA Mutation Mix v2) carrying different TP53 mutations was prepared to use as positive control (Table 3). Sensitivity testing was initially performed starting from 10 ng of this cfDNA of each Seraseq^{®} ctDNA Mutation Mix v2. Samples with different allelic frequency (AF) were used: AF1%, 0.5%, 0.25%, 0.1% and 0%. Specifically 5 different TP53 hotspots were analysed: p.R175H (c.524 G>A), p.C242Afs*5 (c.723del), p.R273H (c.818G>A), p.R248Q (c.743G>A), p.S90Pfs*33 (c.267del). The sequencing overview including reads, coverage, and uniformity of the read coverage distribution is shown in Table 3. In particular, table 3 shows a summary of the starting conditions of the analysis and results. The columns indicates (from left to right): first column Seraseq^{®} samples, AF=allelic frequency, WT=wild type; second and third column: the amount of control DNA and amount of library obtained after amplification of DNA. Fourth column: amount of library used for each sequencing assay. Fifth column: reads (mean reads coverage) obtained after sequencing of each Seraseq^{®}DNA analyzed in duplicate (sample 1 and sample2). Sixth column: TP53 gene variants investigated. Seventh column is indicated the total read coverage (Tot Read Cov) and the coverage for the specific alteration (Alt Read Cov) and the percentage of AF detected for the specific alteration for each sample (Sample 1 and Sample 2).

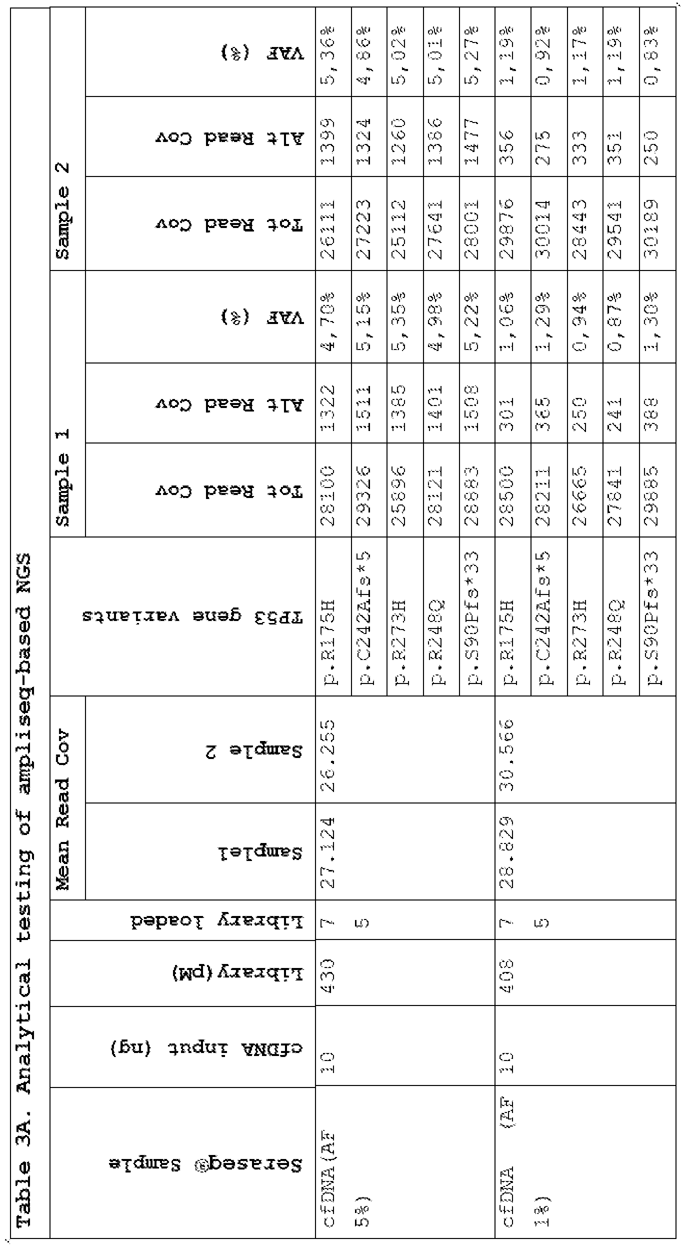

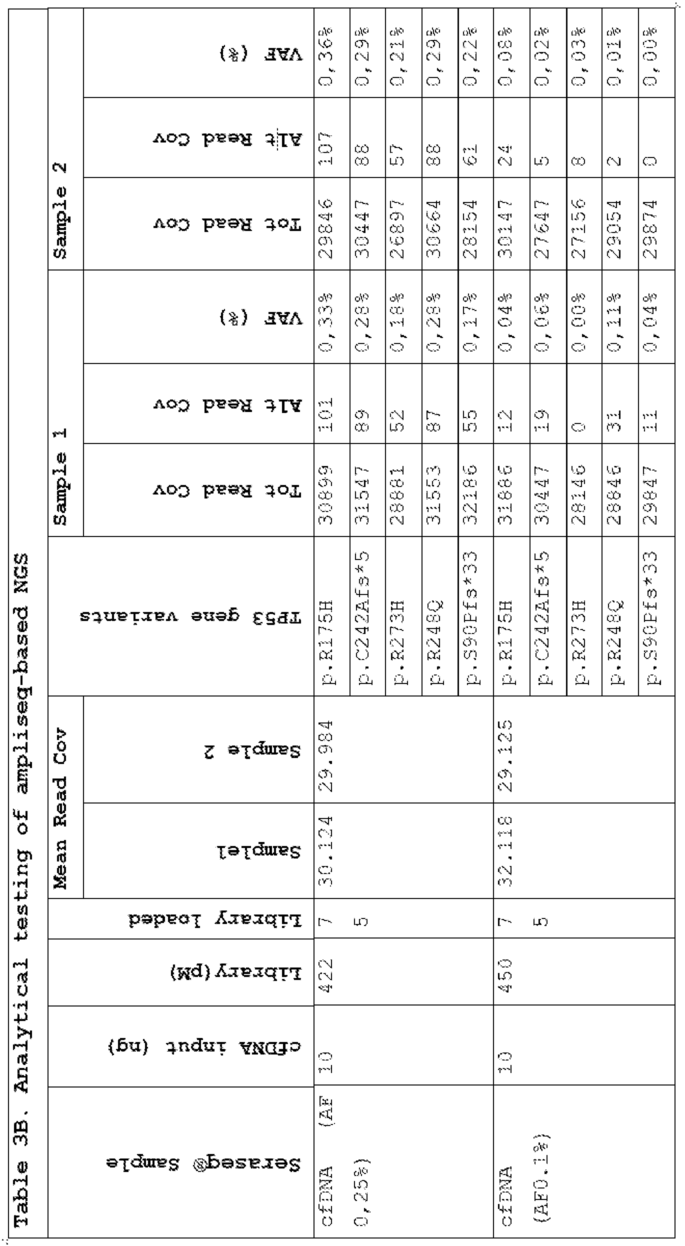

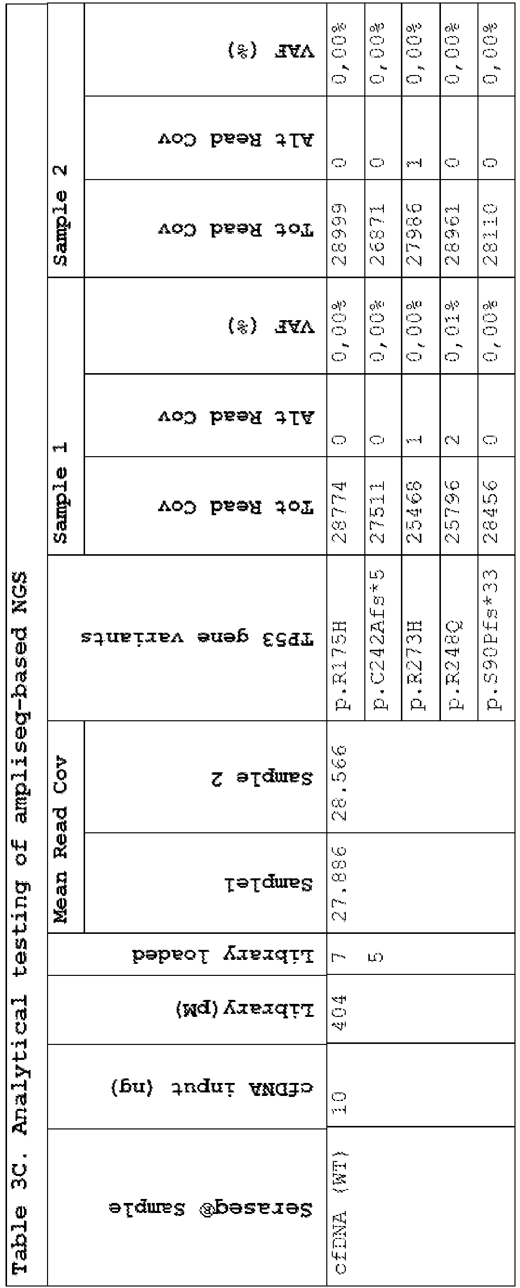

Each specimen underwent an average of 4.88 million sequencing reads after quality filtering. The average of percent (%) assigned amplicon reads in cfDNA was 98.1 (92.4-99.1). Average coverage depth was 29.058 ± 1818 (26.255- 32118). The percent of 12.000x amplicon coverage in total specimens was 93.5% (91.7- 99.0). The ampliseq-based NGS detected all TP53 mutations down to the 0.25% allele frequency with high concordance between the measured allele frequencies with those expected for each reference cfDNAs (Table 3). No false positive call were observed with the test cfDNA containing wild type TP53 gene (0% mutated allele frequency) only, even when the VCF was visualized on IGV software.

In addition, 18 HNSCC tumor biopsies (9 frozen and 9 matched FFPE samples), and 23 matched blood samples (8 pre-surgery and 14 matched post-surgery blood draws) were assessed by H&N chip and dPCR and the results of the two technologies compared. Based on NGS results a specific dPCR assay was designed for 8 different mutations of TP53.

Using H&N panel, TP53 somatic mutations were detected in 16/18 samples on ctDNA and in 3/23 blood samples on cfDNA (Table 4) while the remaining 2 tissues and 20 plasma samples were WT for all mutation exanimated. All of these mutations were validated by dPCR with high level of concordance between the two methods. The validation study with dPCR on tissue and plasma samples is reported in Table 4. In particular, the columns indicate (from left to right): first column: the ID of patients; second column: the number of tissue samples analyzed; third column: the origin of analysed DNA (tumor DNA, or tDNA); Fourth column: the mutation detected with NGS; Fifth column: AF% of the NGS-detected alteration; Sixth column: the mutation detected by dPCR; Seventh column: AF% of the dPCR-detected alteration; eighth column: ID of patients; ninth column: the number of tissue samples analyzed; tenth columns: the origin of analysed DNA (cell free DNA, cfDNA); eleventh column: the mutation detected with NGS; twelfth column: AF% of the NGS-detected alteration;

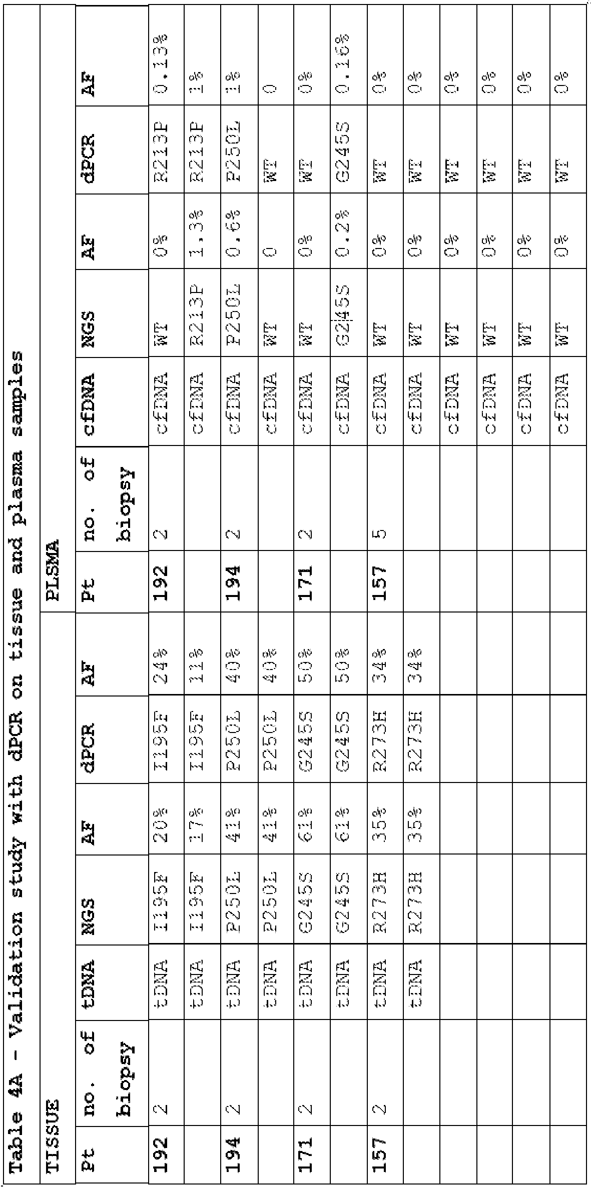

thirteenth column: the mutation detected by dPCR; fourteenth column: AF% of the dPCR-detected alteration.

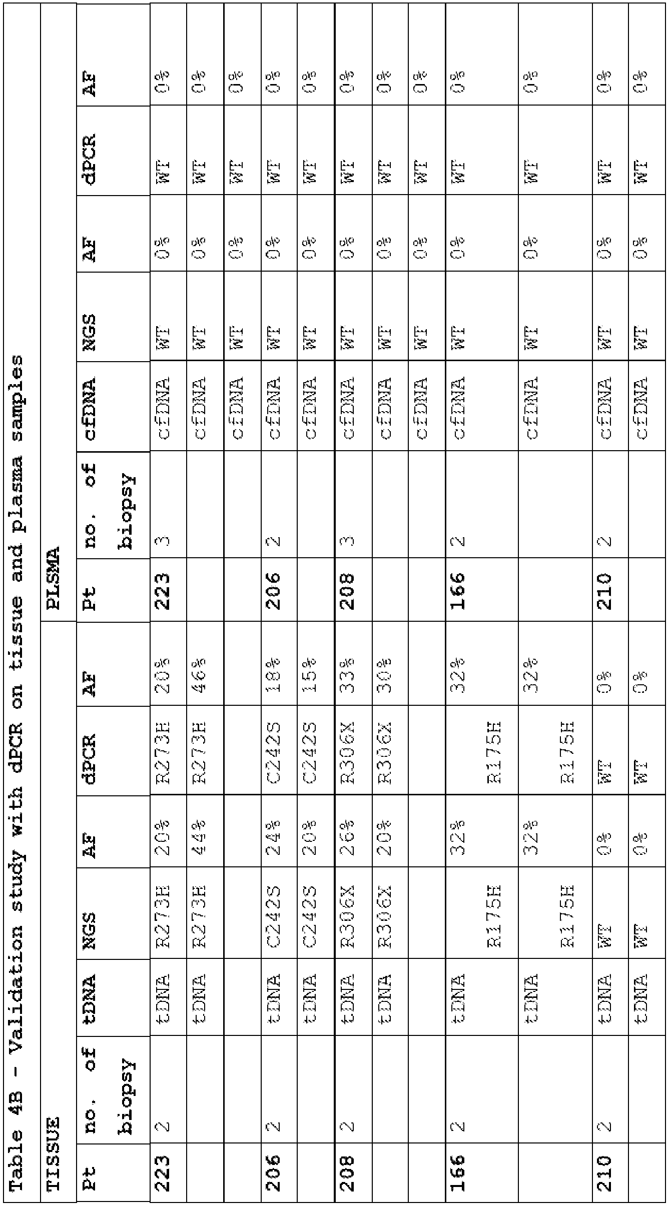

Remarkably, variant call VAFs were concordantly assigned in the double NGS/dPCR setting in both tDNA (VAF from 11% to 61%) and cfDNA (VAF from 0.2% to 3%) (Table 4).

At an expected VAF of ≥0.25%, the present assay detection of SNVs in both cfDNA and tDNA samples exhibited 100% with dPCR. The assay showed a sensitivity of 100% (95% Cl, 82.35% to 100.00%), specificity of 100% (95% Cl, 98.81% to 100.00%), and accuracy of 100% (95% Cl, 98.88% to 100.00%). As the VAF decreased from 0.1%, NGS detected only one false-negative calls in cfDNA (Table 4).

Table 5 shows the performance of the chip in terms of sensitivity, specificity and accuracy.

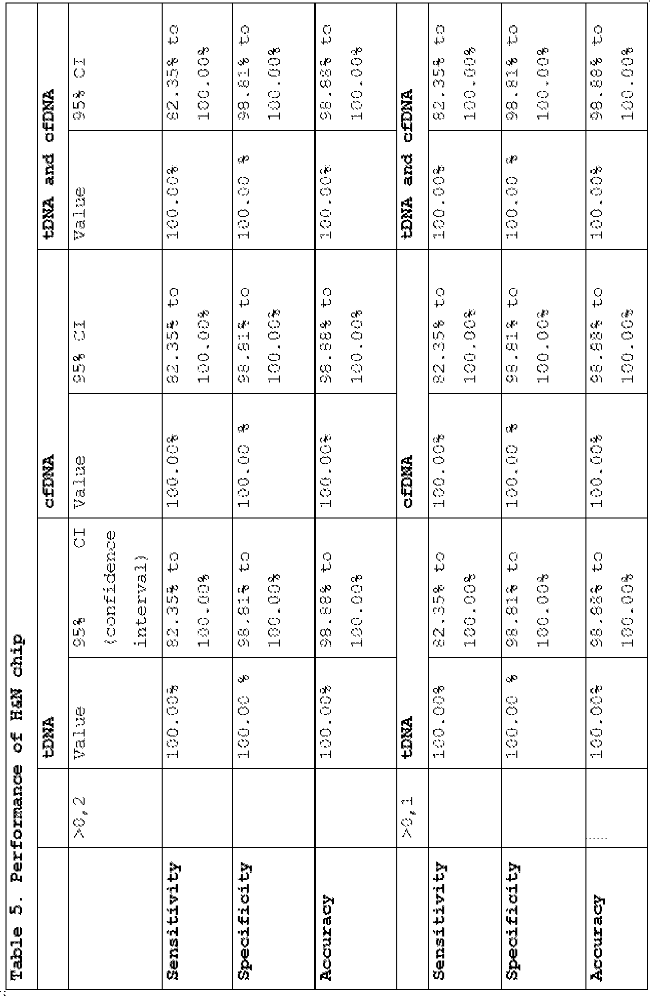

### H&N chip detected mutations in >90% of HNSCC patients

Eighty-nine fresh tumor tissues from a subgroup of HNSCC patients from IRE cohort (9-10) has been analyzed by H&N chip. The custom H&N chip for NGS designed to detect the most frequently mutated genes in tDNA from HNSCC patients (*TP53, FAT1* and *CDKN2A*) (Table 5) identified 145 hits comprising 140 distinct aberrations in the tissues from 81/89 (91%) patients, with frequency of mutation ranging from 2.6 to 81% (Table 6).

Table 6 shows the percentage of TP53, FAT1 and CDKN2A mutations from 89 tDNA in comparison to TCGA public dataset (12).

| ***Table 6. Percentage of TP53, FAT1 and CDKN2A mutations from 89 tDNA*** | | |
|---|---|---|
| | TCGA (530 samples) | IRE (89 samples) |
| TP53 | 72% | 77,5% |
| FAT1 | 23% | 24,6% |
| CDKN2A | 22% | 22,5% |

Table 7 shows the mutations found in the patients. In particular, the table reports the following information: the ID code of the patients, the chromosomal position of the mutation (Locus), the name of the gene on which the mutation was found, the specific mutation (COD.), the frequency of the mutation found (expressed by the ratio between the percentage of the mutated fraction and the percentage of the wild type fraction), the type of mutation (e.g. missense, nonsense etc), the changed base (Genotype), the reference base (Ref), the codon affected by the mutation (Coding)and the aminoacid changed in the sequence of the protein. In particular, table 7 shows the results of tissue sample analysis. The columns indicate (from left to right): first column: ID of patients; second column: gene implicated in the mutation; third column: mutated protein; Fourth column: AF% of the mutation; Fifth column: type of mutation; Sixth column: base changed; Seventh column: base of the reference; eighth column: position and kind of DNA alteration.

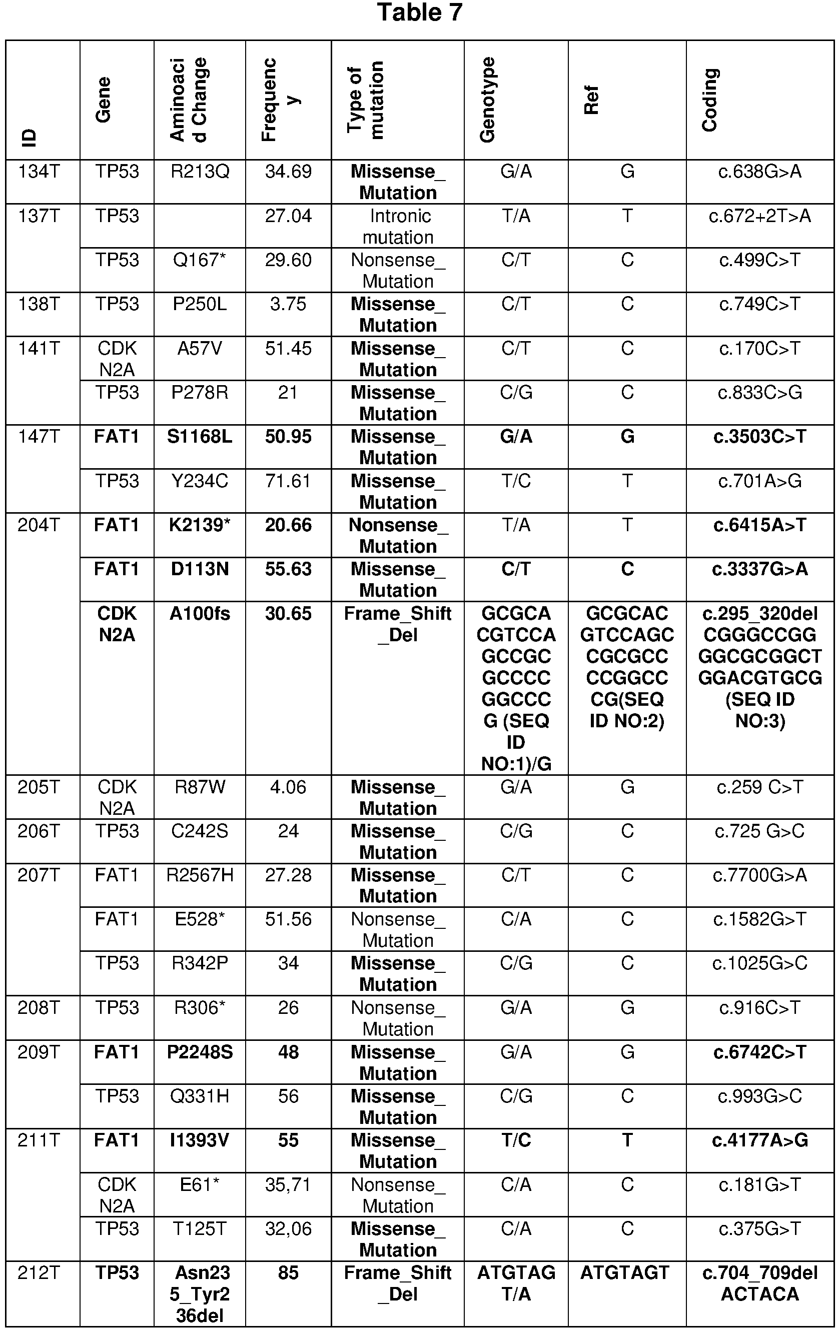

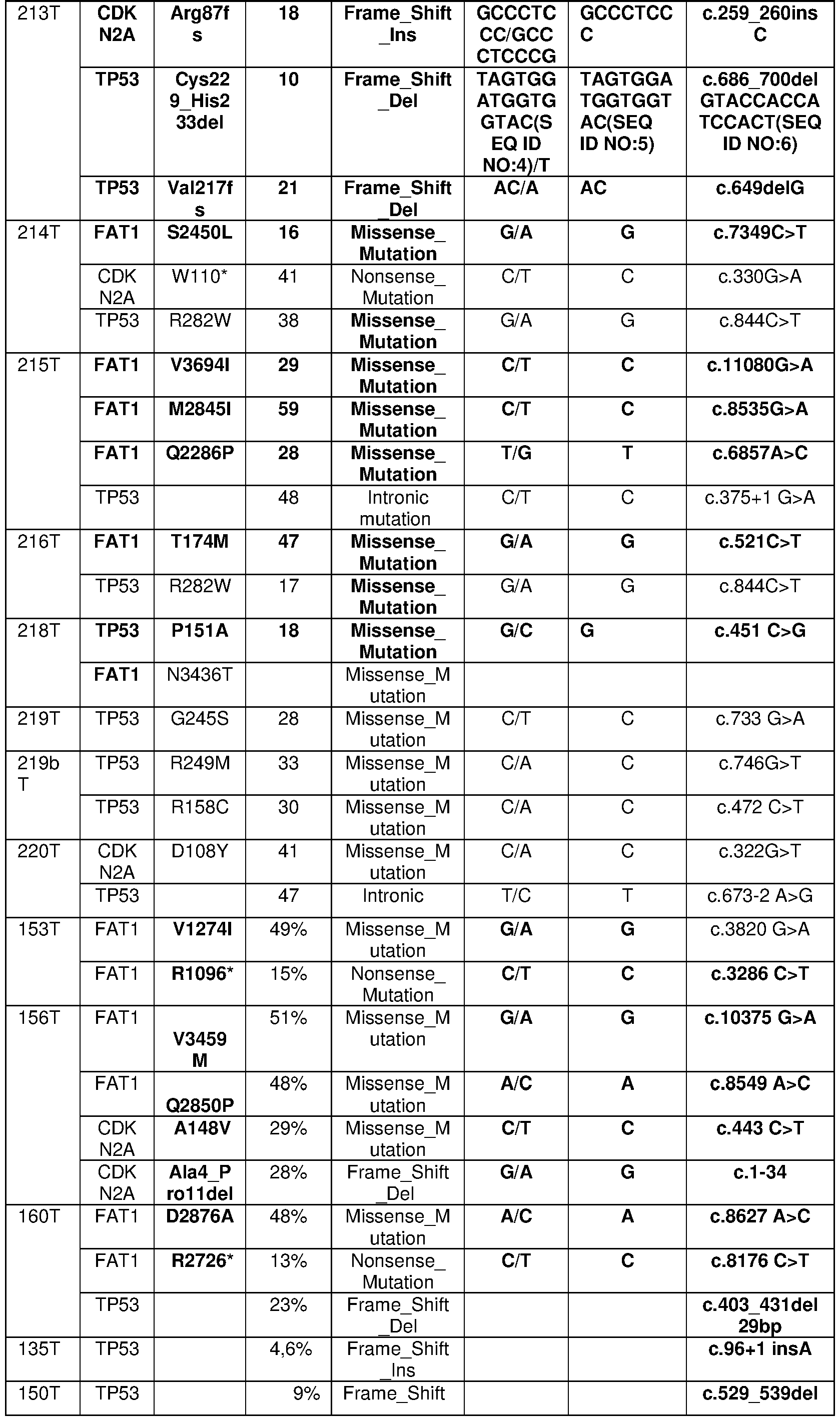

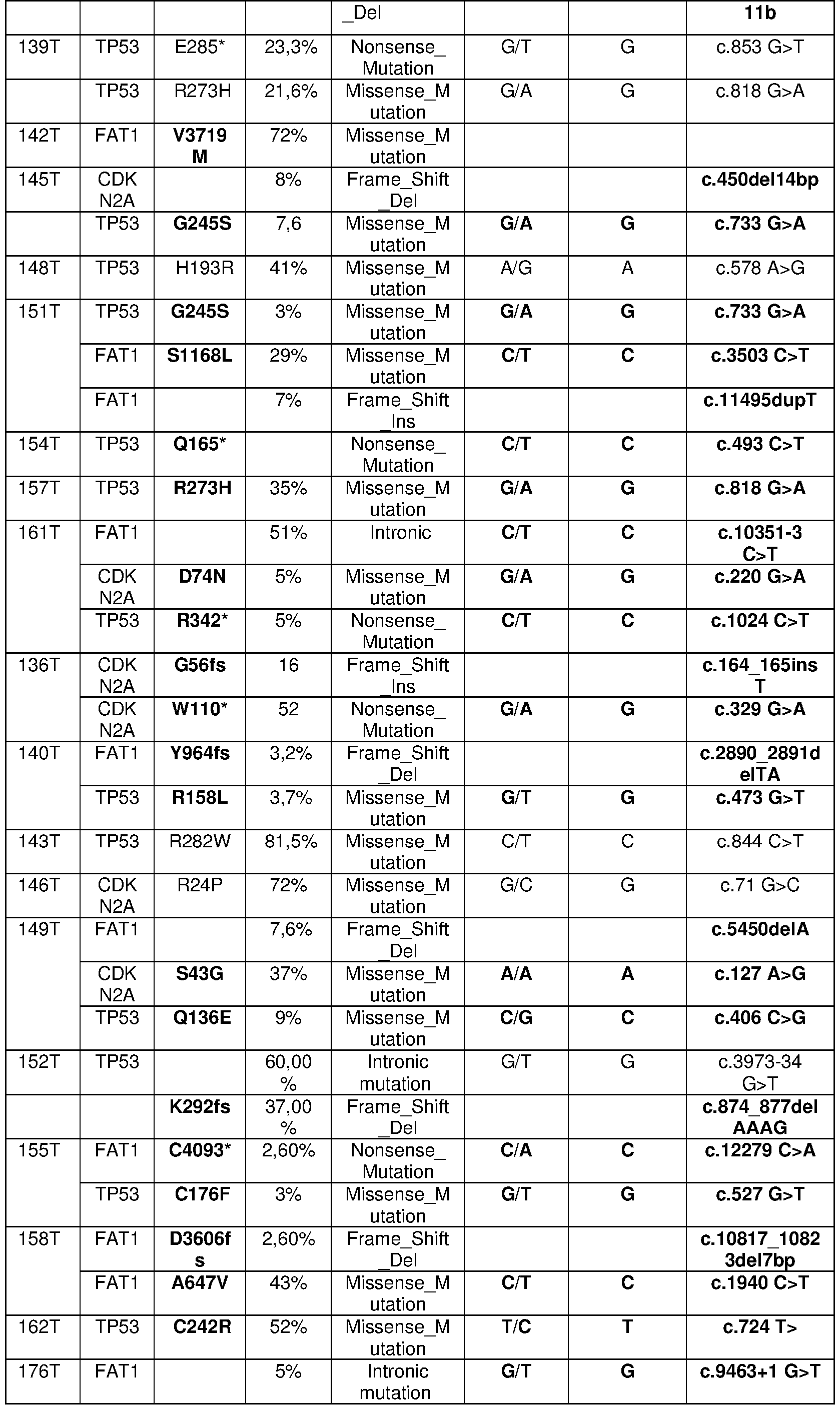

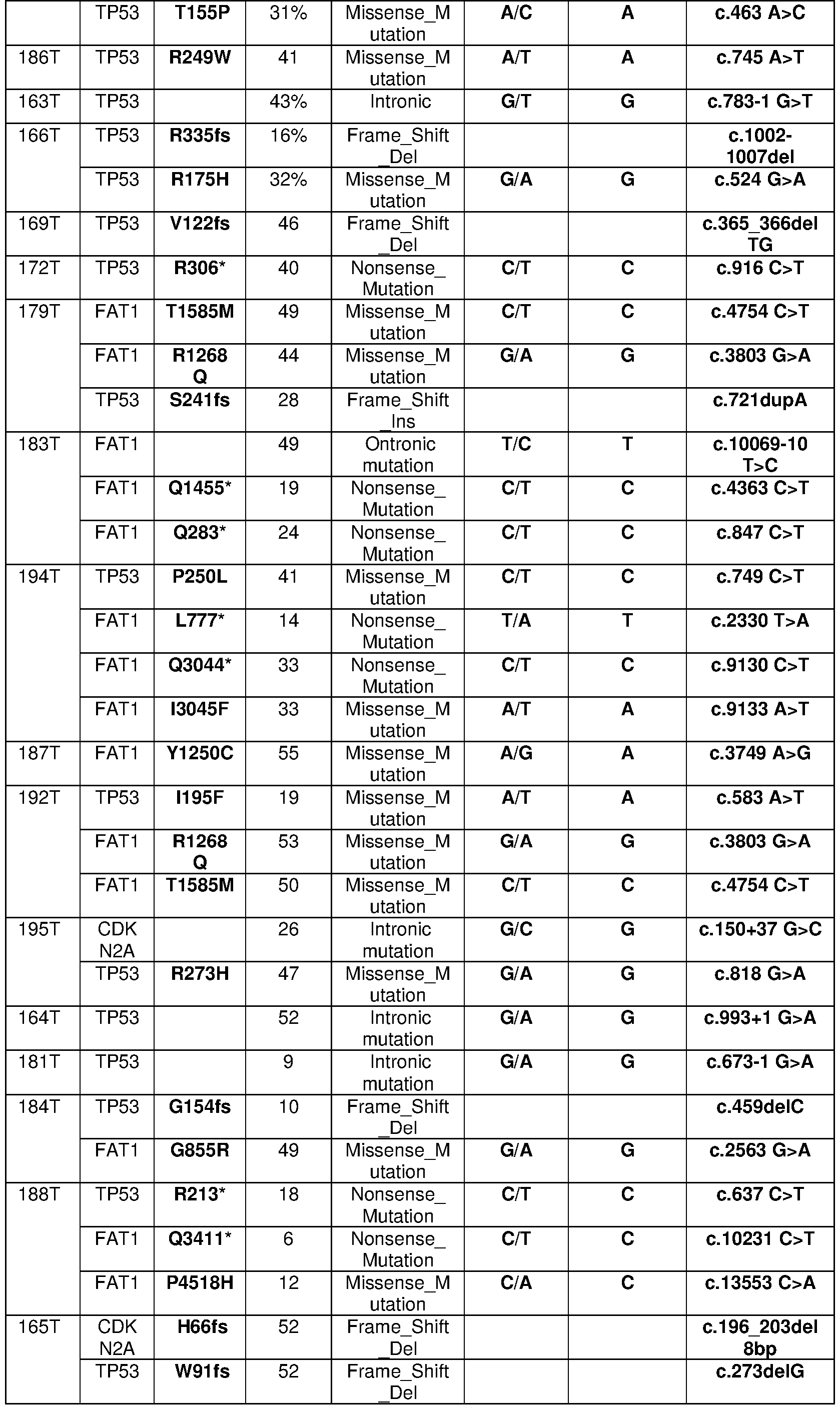

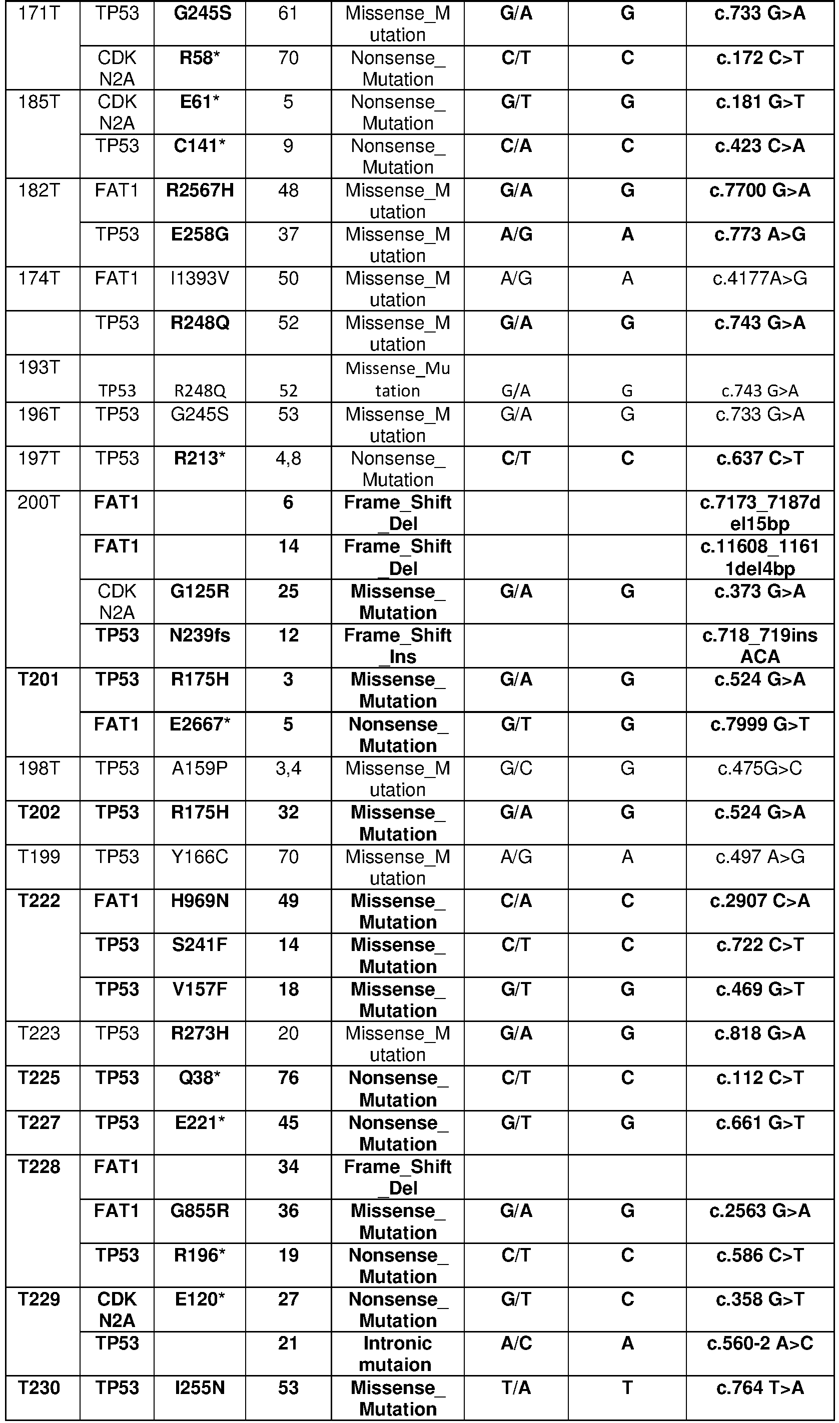

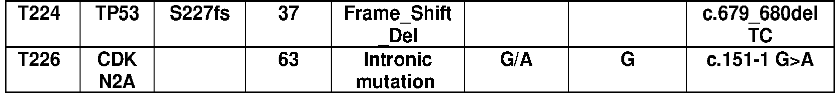

Table 8 shows the percentage of mutations detected with Oncomine Tissue^{™} chip from 89 tDNA.

| ***Tale 8. Percentage of mutations detected with Oncomine tissue^{™} chip from 89 tDNA*** | |
|---|---|
| TP53 | 58 (67,4%) |
| CDKN2A | 15 (17,5%) |
| PIK3CA, APC | 4 (4, 6%) |
| FBXW, MLH1, KRAS, NOTCH1 | 2 (2, 3%) |
| PTEN, FGFR1, HRAS, STK11, ESR1, ROS1, ERBB4, AKT1, KIT | 1 (1, 2%) |

As shown in table 8, the percentage of mutations of the analyzed genes in tumor tissues is in agreement with the percentage showed in TCGA HNSCC cohort. Mutations were not evenly distributed: 8/89 (9%) tDNAs were WT, 41/89 (46.1%) displayed one mutation, and 40/89 (44.9%) displayed multiple aberrations. Novel *FAT1* and *CDKN2A* mutations have been identified, which are reported in Table 9.

**Table 9**

| **FAT1 mutations not yet annotated in ClinVar/Cosmic/HNSCC TCGA datasets for HNSCC samples** | |
|---|---|
| IRE | A3544A |
| IRE | A647V |
| IRE | C4093X |
| IRE | D113N |
| IRE | D2876A |
| IRE | D3606fs |
| IRE | E4274G |
| IRE | H969Q |
| IRE | I1393V |
| IRE | I3045F |
| IRE | K2139X |
| IRE | M2845I |
| IRE | P2248S |
| IRE | P4518H |
| IRE | Q1455X |
| IRE | Q2286P |
| IRE | Q283X |
| IRE | Q2850P |
| IRE | Q3044X |
| IRE | Q3411X |
| IRE | S2450L |
| IRE | T174M |
| IRE | V3694I |
| IRE | V3719M |
| IRE | V3459M |
| IRE | Y1250C |
| IRE | Y964fs |
| IRE | c.10351-3 C>T |
| IRE | c.11495dupT |
| IRE | c.11608_11611del4bp |
| IRE | c.5450delA |
| IRE | c.7173_7187del15bp |
| IRE | c.9463+1 G>T |
| IRE | chr4:187534525_187534532 del8bp (intronica) |
| IRE | c.10069-10 T>C |

| CDKN2A mutations not yet annotated in ClinVar/Cosmic/HNSCC TCGA datasets for HNSCC samples | |
|---|---|
| IRE | S43G |
| IRE | c.1-34 G>A |
| IRE | c.151-1 G>A |
| IRE | c.259_260insC |
| IRE | c.450del14bp |
| IRE | E61X |
| IRE | H66fs |

Concordance between data obtained for *TP53* mutations from direct sequencing, dPCR and NGS in a subset of cases was 100%.

Additional validation of data has been performed by the analysis of all 89 tumor tissues using another panel, the Oncomine tissue^{™} chip, including 50 genes cancer-related (among them *TP53* and *CDKN2A,* but not *FAT1*). Although the number of genes included in commercial panel is higher than the number of genes included in the H&N chip, the number of detected mutations is lower than to H&N chip: 81% vs 91% of HNSCC patients is mutated in at least one gene (see table 8).

### H&N chip identified mutations from different kinds of biological materials in liquid biopsy from HNSCC patients

Thirty-three matched plasma samples before surgery have been also analyzed by H&N chip and validated by dPCR (see paragraph "Analytical validation"). Mutations in *TP53, FAT1* and *CDKN2A* genes from ctDNAs have been detected in 30% of plasma samples; these results are in agreement with other similar studies reported in literature for HNSCC (9). In table 10 are summarized the results.

| **Table 10. *Frequency of TP53, FAT1 and CDKN2A mutations of 33 ctDNAs from HNSCC patients*** | | | | |
|---|---|---|---|---|
| | Tissue | Plasma | % of circulating mutations | Concordance tissue/plasma |
| TP53 mut (N, %) | 29 /33 (88%) | 4/33 (12%) | 4/29 (13,8%) | 3/4 (75%) |
| FAT1 mut (N, %) | 7/33 (21%) | 5/33 (15%) | 5/7 (71,4%) | 2/5 (40%) |
| CDKN2A mut (N, %) | 5/33 (15%) | 2/33 (6%) | 2/5 (40%) | 2/2 (100%) |

Interestingly, for *FAT1* and *TP53* genes a discordance between tissues and matched plasma for a few cases was observed, probably due to the heterogeneity nature of tumor. To verify this hypothesis, one of these cases (case 192/41: Male, 54 years, oral cavity tumor (left tongue)) has been deeply examined. TP53 mutations (p.I195F and R213P) has been identified in tissue and matched plasma. The clinical history of the patient comprised a primitive tumor treated by surgery and radiotherapy, then after ten months a recurrence developed, which was treated by chemotherapy. After 4 months the patient died by cancer. To verify the above-mentioned hypothesis, additional FFPE tissues (tumor, resection margin and lymph nodes one positive at histological analysis and one negative) from this case has been analyzed by H&N chip.

The analysis of these additional materials highlighted the presence of R213P TP53 mutation, previously detected only in plasma, also in tumor tissues as well as in lymph nodes, supporting the idea that the piece of fresh tumor previously analyzed probably did not represent all tumoral cell clones (Ganci et al, 2021). Furthermore, the ability of the CHIP to identify mutations in the plasma, even if not concordant with the tissue, could represent a tool that indicates the need to carry out more in-depth genetic analyzes on the patient.

### H&N chip identified mutations in resection margin and lymph nodes histologically tumor-free of HNSCC patients

Finally, this analysis shows the possibility to detect mutations in histologically tumor free tissue as resection margin and negative lymph nodes. Indeed, the resection margin and some representative lymph nodes both histologically negative for the presence of tumor cells of 4 HNSCC cases with poor outcome, have been analysed. In all cases, the presence of TP53 mutations by NGS and dPCR methods has been detected indicating the presence of tumor cells. This data is particularly interesting because, as explained in the background section, one of the main issues in HNSCC management is the high rate of recurrence also in the presence of histologically negative resection margins or negative lymph nodes. This evidence highlights a further potential application of this chip in the clinical setting. Indeed, its use coupled with the histology examination in the resection margin evaluation could greatly improve the probability to detect tumor cells which although they are not detected at histological level, their presence could be relevant in the recurrence insurgence.

### References

1. Ganci F SA, Manciocco V, Spriano G, Fontemaggi G, Blandino G. Radioresistance in Head and Neck Squamous Cell Carcinoma: Possible Molecular Markers for Local Recurrence and New Putative Therapeutic Strategies. 2015. 3-34 p.
2. Barnett GC et al. "Normal tissue reactions to radiotherapy: towards tailoring treatment dose by genotype". Nat Rev Cancer. 2009 Feb;9(2):134-42.
3. van Houten VM, Leemans CR, Kummer JA, Dijkstra J, Kuik DJ, van den Brekel MW, Snow GB, Brakenhoff RH. Molecular diagnosis of surgical margins and local recurrence in head and neck cancer patients: a prospective study. Clin Cancer Res. 2004 Jun 1;10(11):3614-20.
4. Kwon D, Kim B, Shin HC, Kim EJ, Ha SY, Jang KT, Kim ST, Lee J, Kang WK, Park JO, Kim KM. Cancer Panel Assay for Precision Oncology Clinic: Results from a 1-Year Study. Transl Oncol. 2019 Nov;12(11):1488-1495. doi: 10.1016/j.tranon.2019.07.017. Epub 2019 Aug 20.
5. Ming Chen & Hongyu Zhao Next-generation sequencing in liquid biopsy: cancer screening and early detection Human Genomics volume 13, Article number: 34 (2019)
6. Nonaka T, Wong DTW .J . Liquid Biopsy in Head and Neck Cancer: Promises and Challenges. Dent Res. 2018 Jun;97(6):701-708. Circulating tumor DNA as a biomarker and liquid biopsy in head and neck squamous cell carcinoma.
7. Payne K1, Spruce R2, Beggs A3, Sharma N4, Kong A5, Martin T1, Parmar S1, Praveen P1, Nankivell P4, Mehanna H6. Head Neck. 2018 Jul;40(7):1598-1604. doi: 10.1002/hed.25140. Epub 2018 Mar 15
8. Wang Y, Springer S, Mulvey CL, Silliman N, Schaefer J, Sausen M, James N, Rettig EM, Guo T, Pickering CR, Bishop JA, Chung CH, Califano JA, Eisele DW, Fakhry C, Gourin CG, Ha PK, Kang H, Kiess A, Koch WM, Myers JN, Quon H, Richmon JD, Sidransky D, Tufano RP, Westra WH, Bettegowda C, Diaz LA Jr, Papadopoulos N, Kinzler KW, Vogelstein B, Agrawal N. Detection of somatic mutations and HPV in the saliva and plasma of patients with head and neck squamous cell carcinomas. Sci Transl Med. 2015 Jun 24;7(293):293ra104.
9. Ganci F, Sacconi A, Bossel Ben-Moshe N, Manciocco V, Sperduti I, Strigari L, et al. Expression of TP53 mutation-associated microRNAs predicts clinical outcome in head and neck squamous cell carcinoma patients. Annals of oncology : official journal of the European Society for Medical Oncology / ESMO 2013;24(12):3082-8.
10. Ganci F, Sacconi A, Manciocco V, Covello R, Benevolo M, Rollo F, Strano S, Valsoni S, Bicciato S, Spriano G, Muti P, Fontemaggi G, Blandino G. Altered peritumoral microRNA expression predicts head and neck cancer patients with a high risk of recurrence. Mod Pathol. 2017 Oct;30(10):1387-1401.
11. Perdomo S, Avogbe PH, Foil M, Abedi-Ardekani B, Facciolla VL, Anantharaman D, Chopard P, Calvez-Kelm FL, Vilensky M, Polesel J, Holcatova I, Simonato L, Canova C, Lagiou P, McKay JD, Brennan P. Circulating tumor DNA detection in head and neck cancer: evaluation of two different detection approaches. Oncotarget. 2017 Aug 7;8(42):72621-72632.
12. Comprehensive genomic characterization of head and neck squamous cell carcinomas. Nature 2015;517(7536):576-82 doi 10.1038/nature14129.

## Claims

1. Method for the *in vitro* diagnosis of head and neck cancer, said method comprising detecting, in a biological sample, a mutation in genes consisting of TP53, CDKN2A and FAT1 genes, and, optionally, also in PIK3CA gene, wherein the presence of at least one mutation in one, two or all of said genes in comparison to the respective wild type sequence indicates the presence of head and neck cancer, wherein the method does not comprise detecting a mutation or other aberrations in other genes.

2. Method according to claim 1, wherein the presence of a total of at least two mutations in said one, two or all of said genes in comparison to the respective wild type sequence indicates the presence of:
head and neck cancer which is a immunotherapy responsive head and neck cancer, and/or
head and neck cancer in advanced stage,
wherein said at least two mutations are present in the same gene or in different genes among said one, two or all of said genes.

3. Method according to anyone of claims 1-2, wherein detecting a mutation is carried out on the entire coding regions of TP53, CDKN2A and FAT1 genes.

4. Method according to anyone of claims 1-3, wherein said biological sample is chosen from the group consisting of a liquid biological sample, such as a liquid biopsy, or a tissue sample, wherein said biological sample is fresh, frozen or formalin-fixed paraffin-embedded.

5. Method according to claim 4, wherein said liquid biopsy is chosen from the group consisting of plasma sample or saliva sample, whereas said tissue sample is chosen from the group consisting of a tumor sample, lymph node sample or a resection margin sample.

6. Method according to anyone of claims 1-5, wherein said method is carried out on both a liquid sample and a tissue sample.

7. Method according to anyone of claims 1-6, said method comprising the following steps:
a) extracting DNA from a biological sample;
b) sequencing all of TP53, CDKN2A and FAT1 genes; and
c) detecting mutations in said genes by comparing each sequence of said genes obtained in step b) with the respective wild type sequence.

8. Method according to any one of claims 1-6, wherein the method is carried out by Next Generation Sequencing technologies.

9. Method according to any one of claims 1-8, said method further comprising detecting the following mutations in PIK3CA gene, said mutations having the following COSMIC_id numbers:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
769
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306

10. Kit for the *in vitro* diagnosis of head and neck cancer, said kit comprising suitable reagents for sequencing all TP53, CDKN2A and FAT1 genes and, optionally, also PIK3CA gene, and detecting mutations in said genes in comparison to the respective wild type sequence, whereas the kit does not comprise any reagent for sequencing other genes.

11. Kit according to claim 10, wherein said suitable reagents for sequencing TP53, CDKN2A and FAT1 genes comprise primers for the amplification of said genes, wherein
for the amplification of FAT1 gene, each primer pair has respectively the following initial position of forward primer and final position of reverse primer:
187509686, 187509848
187509780, 187509957
187509922, 187510077
187509985, 187510157
187510068, 187510242
187510198, 187510363
187510240, 187510402
187510358, 187510528
187516739, 187516905
187516839, 187517007
187516950, 187517116
187517558, 187517725
187517686, 187517856
187517722, 187517895
187517847, 187518005
187517899, 187518066
187518000, 187518159
187518082, 187518255
187518147, 187518315
187518290, 187518451
187518790, 187518955
187518844, 187519010
187519017, 187519169
187519125, 187519285
187519235, 187519405
187521006, 187521177
187521066, 187521226
187521171, 187521342
187521242, 187521412
187521376, 187521545
187521496, 187521665
187522275, 187522435
187522395, 187522567
187522457, 187522627
187523994, 187524140
187524081, 187524248
187524279, 187524427
187524386, 187524526
187524421, 187524566
187524522, 187524666
187524564, 187524737
187524660, 187524834
187524748, 187524922
187524831, 187524973
187524920, 187525063
187524986, 187525139
187525062, 187525178
187525498, 187525664
187525619, 187525786
187527123, 187527289
187527218, 187527393
187527324, 187527496
187530280, 187530448
187530335, 187530508
187530870, 187531020
187530976, 187531145
187531040, 187531203
187532465, 187532639
187532511, 187532682
187532635, 187532805
187532720, 187532886
187532798, 187532940
187534199, 187534339
187534304, 187534429
187534332, 187534502
187534438, 187534606
187535251, 187535403
187535344, 187535503
187535400, 187535567
187538056, 187538192
187538141, 187538305
187538188, 187538352
187538783, 187538918
187538869, 187539025
187538912, 187539080
187539017, 187539189
187539078, 187539239
187539190, 187539350
187539232, 187539400
187539355, 187539504
187539394, 187539545
187539501, 187539622
187539540, 187539701
187539627, 187539764
187539699, 187539849
187539759, 187539920
187539840, 187540016
187539911, 187540065
187540015, 187540181
187540073, 187540223
187540184, 187540347
187540236, 187540403
187540345, 187540484
187540400, 187540524
187540481, 187540606
187540538, 187540679
187540598, 187540772
187540687, 187540831
187540770, 187540913
187540830, 187540956
187540925, 187541089
187541016, 187541184
187541085, 187541255
187541183, 187541352
187541254, 187541424
187541348, 187541504
187541418, 187541580
187541507, 187541669
187541577, 187541726
187541666, 187541809
187541724, 187541893
187541813, 187541942
187541887, 187542026
187541943, 187542110
187542025, 187542176
187542120, 187542276
187542186, 187542360
187542279, 187542400
187542353, 187542509
187542400, 187542562
187542504, 187542668
187542558, 187542717
187542661, 187542813
187542760, 187542906
187542806, 187542967
187549198, 187549361
187549316, 187549490
187549385, 187549556
187549591, 187549765
187549720, 187549886
187549777, 187549937
187549890, 187550054
187554804, 187554976
187554897, 187555061
187557084, 187557247
187557176, 187557344
187557249, 187557410
187557365, 187557537
187557616, 187557791
187557747, 187557889
187557818, 187557984
187557903, 187558071
187557985, 187558149
187560789, 187560948
187560877, 187561020
187584406, 187584550
187584482, 187584647
187584544, 187584715
187584650, 187584817
187627676, 187627834
187627720, 187627886
187627828, 187627971
187627889, 187628050
187627968, 187628133
187628051, 187628214
187628126, 187628278
187628217, 187628366
187628288, 187628462
187628360, 187628529
187628459, 187628623
187628528, 187628691
187628623, 187628792
187628696, 187628862
187628784, 187628927
187628863, 187628989
187628920, 187629077
187629015, 187629179
187629069, 187629237
187629181, 187629298
187629229, 187629396
187629305, 187629458
187629390, 187629561
187629454, 187629600
187629553, 187629694
187629606, 187629728
187629686, 187629836
187629785, 187629925
187629830, 187630003
187629924, 187630093
187629997, 187630171
187630090, 187630246
187630182, 187630351
187630276, 187630408
187630355, 187630518
187630437, 187630574
187630516, 187630682
187630616, 187630785
187630687, 187630853
187630782, 187630954
187630858, 187631029
for the amplification of CDKN2A gene, each primer pair has respectively the following initial position of forward primer and final position of reverse primer:
21968149, 21968274
21968651, 21968801
21970835, 21971009
21970962, 21971143
21971106, 21971273
21974425, 21974592
21974542, 21974709
21974650, 21974814
21994109, 21994285
21994241, 21994398
for the amplification of TP53 gene, each primer pair has respectively the following initial position of forward primer and final position of reverse primer:
7572830, 7573002
7572907, 7573051
7573856, 7574026
7573989, 7574156
7576473, 7576588
7576544, 7576709
7576807, 7576971
7576927, 7577092
7577006, 7577176
7577097, 7577256
7577375, 7577531
7577486, 7577639
7578120, 7578282
7578239, 7578398
7578281, 7578453
7578403, 7578580
7579230, 7579398
7579327, 7579505
7579486, 7579616
7579596, 7579770
7579737, 7579874
7579817, 7579988
wherein said positions refer to the hg19 human reference genome.

12. Kit according to anyone of claims 10-11, said kit further comprises primers for detecting the following mutations in PIK3CA gene, said mutations having the following COSMIC_id numbers:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
769
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306

13. Kit according to anyone of claims 10-12, said kit comprising or consisting of a chip that comprises the primers defined in claims 11 and 12.

## Patentansprüche

1. Verfahren zur in-vitro-Diagnose von Kopf- und Halskrebs, wobei das Verfahren den Nachweis einer Mutation in Genen, die aus den Genen TP53, CDKN2A und FAT1 bestehen, und gegebenenfalls auch im PIK3CA-Gen, in einer biologischen Probe umfasst, wobei das Vorhandensein mindestens einer Mutation in einem, zwei oder allen Genen im Vergleich zur jeweiligen Wildtyp-Sequenz das Vorhandensein von Kopf- und Halskrebs anzeigt, wobei das Verfahren nicht den Nachweis einer Mutation oder anderer Aberrationen in anderen Genen umfasst.

2. Verfahren nach Anspruch 1, wobei das Vorhandensein von insgesamt mindestens zwei Mutationen in dem einen, zwei oder allen Genen im Vergleich zur jeweiligen Wildtyp-Sequenz das Vorhandensein von anzeigt: kopf- und Halskrebs, der auf eine Immuntherapie anspricht, und/oder kopf- und Halskrebs im fortgeschrittenen Stadium, wobei die mindestens zwei Mutationen in demselben Gen oder in verschiedenen Genen unter den ein, zwei oder allen Genen vorhanden sind.

3. Verfahren nach einem der Ansprüche 1-2, wobei der Nachweis einer Mutation an den gesamten kodierenden Regionen der Gene TP53, CDKN2A und FAT1 durchgeführt wird.

4. Verfahren nach einem der Ansprüche 1-3, wobei die biologische Probe aus der Gruppe ausgewählt wird, die aus einer flüssigen biologischen Probe, wie z. B. einer flüssigen Biopsie, oder einer Gewebeprobe besteht, wobei die biologische Probe frisch, gefroren oder formalinfixiert und in Paraffin eingebettet ist.

5. Verfahren nach Anspruch 4, wobei die Flüssigbiopsie aus der Gruppe ausgewählt wird, die aus einer Plasmaprobe oder Speichelprobe besteht, während die Gewebeprobe aus der Gruppe ausgewählt wird, die aus einer Tumorprobe, einer Lymphknotenprobe oder einer Resektionsrandprobe besteht.

6. Verfahren nach einem der Ansprüche 1-5, wobei das Verfahren sowohl an einer Flüssigkeitsprobe als auch an einer Gewebeprobe durchgeführt wird.

7. Verfahren nach einem der Ansprüche 1-6, wobei das Verfahren die folgenden Schritte umfasst:
a) Extraktion von DNA aus einer biologischen Probe;
b) Sequenzierung aller TP53-, CDKN2A- und FAT1-Gene; und
c) Nachweis von Mutationen in den genannten Genen durch Vergleich jeder in Schritt b) erhaltenen Sequenz der genannten Gene mit der jeweiligen Wildtyp-Sequenz.

8. Verfahren nach einem der Ansprüche 1-6, wobei das Verfahren mittels Next Generation Sequencing Technologien durchgeführt wird.

9. Verfahren nach einem der Ansprüche 1-8, wobei das Verfahren ferner den Nachweis der folgenden Mutationen im PIK3CA-Gen umfasst, wobei die Mutationen die folgenden COSMIC_id-Nummern aufweisen:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306

10. Kit für die in-vitro-diagnose von Kopf- und Halskrebs, wobei das Kit geeignete Reagenzien für die Sequenzierung aller TP53-, CDKN2A- und FAT1-Gene und gegebenenfalls auch des PIK3CA-Gens und den Nachweis von Mutationen in diesen Genen im Vergleich zur jeweiligen Wildtyp-Sequenz umfasst, während das Kit kein Reagenz für die Sequenzierung anderer Gene umfasst.

11. Kit nach Anspruch 10, wobei die geeigneten Reagenzien für die Sequenzierung von TP53-, CDKN2A- und FAT1-Genen Primer für die Amplifikation dieser Gene umfassen, wobei für die Amplifikation des FAT1-Gens hat jedes Primerpaar die folgende Anfangsposition des Vorwärtsprimers und die Endposition des Rückwärtsprimers:
187509686, 187509848
187509780, 187509957
187509922, 187510077
187509985, 187510157
187510068, 187510242
187510198, 187510363
187510240, 187510402
187510358, 187510528
187516739, 187516905
187516839, 187517007
187516950, 187517116
187517558, 187517725
187517686, 187517856
187517722, 187517895
187517847, 187518005
187517899, 187518066
187518000, 187518159
187518082, 187518255
187518147, 187518315
187518290, 187518451
187518790, 187518955
187518844, 187519010
187519017, 187519169
187519125, 187519285
187519235, 187519405
187521006, 187521177
187521066, 187521226
187521171, 187521342
187521242, 187521412
187521376, 187521545
187521496, 187521665
187522275, 187522435
187522395, 187522567
187522457, 187522627
187523994, 187524140
187524081, 187524248
187524279, 187524427
187524386, 187524526
187524421, 187524566
187524522, 187524666
187524564, 187524737
187524660, 187524834
187524748, 187524922
187524831, 187524973
187524920, 187525063
187524986, 187525139
187525062, 187525178
187525498, 187525664
187525619, 187525786
187527123, 187527289
187527218, 187527393
187527324, 187527496
187530280, 187530448
187530335, 187530508
187530870, 187531020
187530976, 187531145
187531040, 187531203
187532465, 187532639
187532511, 187532682
187532635, 187532805
187532720, 187532886
187532798, 187532940
187534199, 187534339
187534304, 187534429
187534332, 187534502
187534438, 187534606
187535251, 187535403
187535344, 187535503
187535400, 187535567
187538056, 187538192
187538141, 187538305
187538188, 187538352
187538783, 187538918
187538869, 187539025
187538912, 187539080
187539017, 187539189
187539078, 187539239
187539190, 187539350
187539232, 187539400
187539355, 187539504
187539394, 187539545
187539501, 187539622
187539540, 187539701
187539627, 187539764
187539699, 187539849
187539759, 187539920
187539840, 187540016
187539911, 187540065
187540015, 187540181
187540073, 187540223
187540184, 187540347
187540236, 187540403
187540345, 187540484
187540400, 187540524
187540481, 187540606
187540538, 187540679
187540598, 187540772
187540687, 187540831
187540770, 187540913
187540830, 187540956
187540925, 187541089
187541016, 187541184
187541085, 187541255
187541183, 187541352
187541254, 187541424
187541348, 187541504
187541418, 187541580
187541507, 187541669
187541577, 187541726
187541666, 187541809
187541724, 187541893
187541813, 187541942
187541887, 187542026
187541943, 187542110
187542025, 187542176
187542120, 187542276
187542186, 187542360
187542279, 187542400
187542353, 187542509
187542400, 187542562
187542504, 187542668
187542558, 187542717
187542661, 187542813
187542760, 187542906
187542806, 187542967
187549198, 187549361
187549316, 187549490
187549385, 187549556
187549591, 187549765
187549720, 187549886
187549777, 187549937
187549890, 187550054
187554804, 187554976
187554897, 187555061
187557084, 187557247
187557176, 187557344
187557249, 187557410
187557365, 187557537
187557616, 187557791
187557747, 187557889
187557818, 187557984
187557903, 187558071
187557985, 187558149
187560789, 187560948
187560877, 187561020
187584406, 187584550
187584482, 187584647
187584544, 187584715
187584650, 187584817
187627676, 187627834
187627720, 187627886
187627828, 187627971
187627889, 187628050
187627968, 187628133
187628051, 187628214
187628126, 187628278
187628217, 187628366
187628288, 187628462
187628360, 187628529
187628459, 187628623
187628528, 187628691
187628623, 187628792
187628696, 187628862
187628784, 187628927
187628863, 187628989
187628920, 187629077
187629015, 187629179
187629069, 187629237
187629181, 187629298
187629229, 187629396
187629305, 187629458
187629390, 187629561
187629454, 187629600
187629553, 187629694
187629606, 187629728
187629686, 187629836
187629785, 187629925
187629830, 187630003
187629924, 187630093
187629997, 187630171
187630090, 187630246
187630182, 187630351
187630276, 187630408
187630355, 187630518
187630437, 187630574
187630516, 187630682
187630616, 187630785
187630687, 187630853
187630782, 187630954
187630858, 187631029
für die Amplifikation des CDKN2A-Gens hat jedes Primerpaar die folgende Anfangsposition des Vorwärtsprimers und die Endposition des Rückwärtsprimers:
21968149, 21968274
21968651, 21968801
21970835, 21971009
21970962, 21971143
21971106, 21971273
21974425, 21974592
21974542, 21974709
21974650, 21974814
21994109, 21994285
21994241, 21994398
für die Amplifikation des TP53-Gens hat jedes Primerpaar die folgende Anfangsposition des Vorwärtsprimers und die Endposition des Rückwärtsprimers:
7572830, 7573002
7572907, 7573051
7573856, 7574026
7573989, 7574156
7576473, 7576588
7576544, 7576709
7576807, 7576971
7576927, 7577092
7577006, 7577176
7577097, 7577256
7577375, 7577531
7577486, 7577639
7578120, 7578282
7578239, 7578398
7578281, 7578453
7578403, 7578580
7579230, 7579398
7579327, 7579505
7579486, 7579616
7579596, 7579770
7579737, 7579874
7579817, 7579988
wobei sich diese Positionen auf das menschliche Referenzgenom hg19 beziehen.

12. Kit nach einem der Ansprüche 10-11, wobei das Kit ferner Primer zum Nachweis der folgenden Mutationen im PIK3CA-Gen umfasst, wobei die Mutationen die folgenden COSMIC_id-Nummern aufweisen:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
769
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306

13. Kit nach einem der Ansprüche 10-12, wobei der Kit einen Chip umfasst oder aus einem Chip besteht, der die in den Ansprüchen 11 und 12 definierten Primer fenthält.

## Revendications

1. Méthode de diagnostic *in vitro* du cancer de la tête et du cou, comprenant la détection, dans un échantillon biologique, d'une mutation dans les gènes TP53, CDKN2A et FAT1 et, éventuellement, dans le gène PIK3CA, la présence d'au moins une mutation dans un, deux ou tous ces gènes par rapport à la séquence sauvage respective indiquant la présence d'un cancer de la tête et du cou, la méthode ne comprenant pas la détection d'une mutation ou d'autres aberrations dans d'autres gènes.

2. Méthode selon la revendication 1, dans laquelle la présence d'un total d'au moins deux mutations dans un, deux ou tous les gènes par rapport à la séquence de type sauvage respective indique la présence de: cancer de la tête et du cou répondant à l'immunothérapie, et/ou cancer de la tête et du cou à un stade avancé, dans lequel ces deux mutations au moins sont présentes dans le même gène ou dans différents gènes parmi ces un, deux ou tous ces gènes.

3. Méthode selon l'une des revendications 1 et 2, dans laquelle la détection d'une mutation est effectuée sur l'ensemble des régions codantes des gènes TP53, CDKN2A et FAT1.

4. Méthode selon l'une des revendications 1-3, dans laquelle ledit échantillon biologique est choisi dans le groupe constitué d'un échantillon biologique liquide, tel qu'une biopsie liquide, ou d'un échantillon de tissu, dans lequel ledit échantillon biologique est frais, congelé ou fixé au formol et inclus dans la paraffine.

5. Méthode selon la revendication 4, dans laquelle ladite biopsie liquide est choisie dans le groupe constitué d'un échantillon de plasma ou de salive, tandis que ledit échantillon de tissu est choisi dans le groupe constitué d'un échantillon de tumeur, d'un échantillon de ganglion lymphatique ou d'un échantillon de marge de résection.

6. Méthode selon l'une des revendications 1-5, dans laquelle ladite méthode est appliquée à la fois à un échantillon de liquide et à un échantillon de tissu.

7. Méthode selon l'une des revendications 1-6, comprenant les étapes suivantes:
a) l'extraction de l'DNA à partir d'un échantillon biologique;
b) le séquençage de tous les gènes TP53, CDKN2A et FAT1; et
c) la détection des mutations dans lesdits gènes en comparant chaque séquence desdits gènes obtenue à l'étape b) avec la séquence de type sauvage correspondante.

8. Méthode selon l'une des revendications 1-6, dans laquelle la méthode est réalisée à l'aide de technologies de séquençage de nouvelle génération.

9. Méthode selon l'une des revendications 1-8, ladite méthode comprenant en outre la détection des mutations suivantes dans le gène PIK3CA, lesdites mutations ayant les numéros COSMIC_id suivants:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306

10. Kit pour le diagnostic *in vitro* du cancer de la tête et du cou, comprenant des réactifs appropriés pour le séquençage de tous les gènes TP53, CDKN2A et FAT1 et, éventuellement, du gène PIK3CA, et pour la détection des mutations dans ces gènes par rapport à la séquence de type sauvage respective, alors que le kit ne comprend aucun réactif pour le séquençage d'autres gènes.

11. Kit selon la revendication 10, dans lequel lesdits réactifs appropriés pour le séquençage des gènes TP53, CDKN2A et FAT1 comprennent des amorces pour l'amplification desdits gènes, dans lequel pour l'amplification du gène FAT1, chaque paire d'amorces a respectivement la position initiale suivante de l'amorce avant et la position finale de l'amorce arrière:
187509686, 187509848
187509780, 187509957
187509922, 187510077 187509985, 187510157 187510068, 187510242 187510198, 187510363 187510240, 187510402 187510358, 187510528 187516739, 187516905 187516839, 187517007 187516950, 187517116 187517558, 187517725 187517686, 187517856 187517722, 187517895 187517847, 187518005 187517899, 187518066 187518000, 187518159 187518082, 187518255 187518147, 187518315 187518290, 187518451 187518790, 187518955 187518844, 187519010 187519017, 187519169 187519125, 187519285 187519235, 187519405 187521006, 187521177 187521066, 187521226
187521171, 187521342
187521242, 187521412
187521376, 187521545
187521496, 187521665
187522275, 187522435
187522395, 187522567
187522457, 187522627
187523994, 187524140
187524081, 187524248
187524279, 187524427
187524386, 187524526
187524421, 187524566
187524522, 187524666
187524564, 187524737
187524660, 187524834
187524748, 187524922
187524831, 187524973
187524920, 187525063
187524986, 187525139
187525062, 187525178
187525498, 187525664
187525619, 187525786
187527123, 187527289
187527218, 187527393
187527324, 187527496
187530280, 187530448
187530335, 187530508
187530870, 187531020
187530976, 187531145
187531040, 187531203
187532465, 187532639
187532511, 187532682
187532635, 187532805
187532720, 187532886
187532798, 187532940
187534199, 187534339
187534304, 187534429
187534332, 187534502
187534438, 187534606
187535251, 187535403
187535344, 187535503
187535400, 187535567
187538056, 187538192
187538141, 187538305
187538188, 187538352
187538783, 187538918
187538869, 187539025
187538912, 187539080
187539017, 187539189
187539078, 187539239
187539190, 187539350
187539232, 187539400
187539355, 187539504
187539394, 187539545
187539501, 187539622
187539540, 187539701
187539627, 187539764
187539699, 187539849
187539759, 187539920
187539840, 187540016
187539911, 187540065
187540015, 187540181
187540073, 187540223
187540184, 187540347
187540236, 187540403
187540345, 187540484
187540400, 187540524
187540481, 187540606
187540538, 187540679
187540598, 187540772
187540687, 187540831
187540770, 187540913
187540830, 187540956
187540925, 187541089
187541016, 187541184
187541085, 187541255
187541183, 187541352
187541254, 187541424
187541348, 187541504
187541418, 187541580
187541507, 187541669
187541577, 187541726
187541666, 187541809
187541724, 187541893
187541813, 187541942
187541887, 187542026
187541943, 187542110
187542025, 187542176
187542120, 187542276
187542186, 187542360
187542279, 187542400
187542353, 187542509
187542400, 187542562
187542504, 187542668
187542558, 187542717
187542661, 187542813
187542760, 187542906
187542806, 187542967
187549198, 187549361
187549316, 187549490
187549385, 187549556
187549591, 187549765
187549720, 187549886
187549777, 187549937
187549890, 187550054
187554804, 187554976
187554897, 187555061
187557084, 187557247
187557176, 187557344
187557249, 187557410
187557365, 187557537
187557616, 187557791
187557747, 187557889
187557818, 187557984
187557903, 187558071
187557985, 187558149
187560789, 187560948
187560877, 187561020
187584406, 187584550
187584482, 187584647
187584544, 187584715
187584650, 187584817
187627676, 187627834
187627720, 187627886
187627828, 187627971
187627889, 187628050
187627968, 187628133
187628051, 187628214
187628126, 187628278
187628217, 187628366
187628288, 187628462
187628360, 187628529
187628459, 187628623
187628528, 187628691
187628623, 187628792
187628696, 187628862
187628784, 187628927
187628863, 187628989
187628920, 187629077
187629015, 187629179
187629069, 187629237
187629181, 187629298
187629229, 187629396
187629305, 187629458
187629390, 187629561
187629454, 187629600
187629553, 187629694
187629606, 187629728
187629686, 187629836
187629785, 187629925
187629830, 187630003
187629924, 187630093
187629997, 187630171
187630090, 187630246
187630182, 187630351
187630276, 187630408
187630355, 187630518
187630437, 187630574
187630516, 187630682
187630616, 187630785
187630687, 187630853
187630782, 187630954
187630858, 187631029
pour l'amplification du gène CDKN2A, chaque paire d'amorces a respectivement la position initiale suivante de l'amorce avant et la position finale de l'amorce arrière:
21968149, 21968274
21968651, 21968801
21970835, 21971009
21970962, 21971143
21971106, 21971273
21974425, 21974592
21974542, 21974709
21974650, 21974814
21994109, 21994285
21994241, 21994398
pour l'amplification du gène TP53, chaque paire d'amorces a respectivement la position initiale suivante de l'amorce avant et la position finale de l'amorce arrière:
7572830, 7573002
7572907, 7573051
7573856, 7574026
7573989, 7574156
7576473, 7576588
7576544, 7576709
7576807, 7576971
7576927, 7577092
7577006, 7577176
7577097, 7577256
7577375, 7577531
7577486, 7577639
7578120, 7578282
7578239, 7578398
7578281, 7578453
7578403, 7578580
7579230, 7579398
7579327, 7579505
7579486, 7579616
7579596, 7579770
7579737, 7579874
7579817, 7579988
où lesdites positions se réfèrent au génome humain de référence hg19.

12. Kit selon l'une des revendications 10-11, ledit kit comprend en outre des amorces pour détecter les mutations suivantes dans le gène PIK3CA, lesdites mutations ayant les numéros COSMIC_id suivants:
12458
12459
12461
12463
12464
12580
12582
12584
12590
12591
12592
12597
13570
13594
17442
17444
17445
17449
21451
21462
22540
24712
24714
249872
249908
25041
25085
25086
27133
27134
27155
27156
27158
27273
27374
27502
27505
28938
29110
29313
36285
36286
36289
6147
746
754
757
759
760
762
763
764
765
766
767
769
770
771
772
773
774
775
776
777
778
94984
125368
12579
12589
14052
163484
166154
21448
230020
24710
24711
27490
27497
27499
39166
41785
51258
51259
6145
6149
6150
749
750
758
86045
86949
86950
87210
125370
94985
94986
94987
COSM87306

13. Kit selon l'une des revendications 10-12, ledit kit comprenant ou consistant en une puce qui comprend les amorces définies dans les revendications 11 et 12.
